# EUROPEAN PATENT SPECIFICATION

(11) **EP 2 828 658 B1**
(45) Date of publication and mention of the grant of the patent: **14.06.2017**
(21) Application number: 13763754.2
(22) Date of filing: 15.03.2013
(51) Int. Cl.: G01N 33/53

(54) **METHODS OF IDENTIFYING HIT-ANTIBODIES AND PF4 ANTAGONISTS AND CELL LINES FOR USE THEREIN**
VERFAHREN ZUR IDENTIFIKATION VON HIT-ANTIKÖRPERN UND PF4-ANTIKÖRPERN SOWIE ZELLLINIEN ZUR VERWENDUNG DARIN
MÉTHODES D'IDENTIFICATION D'ANTICORPS HIT ET D'ANTAGONISTES PF4 ET LIGNÉES CELLULAIRES UTILISABLES À CET EFFET

(30) Priority: 23.03.2012 US 201261614729 P
(43) Date of publication of application: 28.01.2015
(73) Proprietor: The Trustees of The University of Pennsylvania, Philadelphia, PA 19104 (US); The University Of Birmingham, Birmingham B15 2TT (GB)
(72) Inventor: WATSON, Steve, P., Edgbaston Birmingham B15 2TT (GB); SACHAIS, Bruce, Cherry Hill, NJ 08003 (US); CINES, Douglas, B., Wynnewood, PA 19096 (US)
(74) Representative: Wallis, Naomi Rachel
(86) International application number: PCT/US2013/032100
(87) International publication number: WO 2013/142330

(56) References cited:
- WO-A2-01/16184
- US-A1- 2004 076 954
- US-A1- 2012 040 373
- REILLY M P ET AL: "HEPARIN-INDUCED THROMBOCYTOPENIA/THROMBOSIS IN A TRANSGENIC MOUSE MODEL REQUIRES HUMAN PLATELET FACTOR 4 AND PLATELET ACTIVATION THROUGH FCGAMMARIIA", BLOOD, AMERICAN SOCIETY OF HEMATOLOGY, US, vol. 98, no. 8, 15 October 2001 (2001-10-15), pages 2442-2447, XP001035093, ISSN: 0006-4971, DOI: 10.1182/BLOOD.V98.8.2442
- L. RAUOVA ET AL: "Ultralarge complexes of PF4 and heparin are central to the pathogenesis of heparin-induced thrombocytopenia", BLOOD, vol. 105, no. 1, 1 January 2005 (2005-01-01), pages 131-138, XP055223350, US ISSN: 0006-4971, DOI: 10.1182/blood-2004-04-1544
- L. RAUOVA ET AL: "Role of platelet surface PF4 antigenic complexes in heparin-induced thrombocytopenia pathogenesis: diagnostic and therapeutic implications", BLOOD, vol. 107, no. 6, 15 March 2006 (2006-03-15), pages 2346-2353, XP055223351, US ISSN: 0006-4971, DOI: 10.1182/blood-2005-08-3122
- B. S. SACHAIS ET AL: "Rational design and characterization of platelet factor 4 antagonists for the study of heparin-induced thrombocytopenia", BLOOD, vol. 119, no. 25, 21 June 2012 (2012-06-21), pages 5955-5962, XP055223349, US ISSN: 0006-4971, DOI: 10.1182/blood-2012-01-406801
- Y. LIU ET AL: "Cytokine-mediated regulation of activating and inhibitory Fc receptors in human monocytes", JOURNAL OF LEUKOCYTE BIOLOGY, vol. 77, no. 5, 1 May 2005 (2005-05-01), pages 767-776, XP055223686, US ISSN: 0741-5400, DOI: 10.1189/jlb.0904532
- CHONG ET AL.: 'Increased expression of platelet IgG Fc receptors in immune heparin-induced thrombocytopenia.' BLOOD vol. 81, no. 4, 15 February 1993, pages 988 - 993, XP055163063
- AMIRAL ET AL.: 'Generation of antibodies to heparin-PF4 complexes without thrombocytopenia in patients treated with unfractionated or low-molecular-weight heparin.' AMERICAN JOURNAL OF HEMATOLOGY vol. 52, no. 2, June 1996, pages 90 - 95, XP055163066

## Description

### BACKGROUND OF THE INVENTION

Heparin-induced thrombocytopenia (HIT) and thrombosis (HITT) are serious complications of heparin therapy. HIT occurs in approximately 1% of patients exposed to therapeutic doses of unfractionated heparin for 5-10 days. HITT is a severe prothrombotic disease, with affected individuals having a 20-50% risk of developing new thromboembolic events, and has a mortality rate of about 20% with an additional about 10% of patients requiring amputations or suffering other major morbidity. Since a large number of hospitalized patients are exposed to heparin, HITT is a major iatrogenic cause of morbidity and mortality in this patient population. No specific therapies to treat HITT are reported. Management consists of general measures such as withdrawal of heparin, use of a non-heparin anticoagulant, and supportive care.

PF4 is a 70 amino acid, lysine-rich 7.8 kDa platelet-specific protein that belongs to the CXC (or beta) chemokine subfamily. PF4 is synthesized by megakaryocytes and comprises 2-3% of the total released protein in mature platelets. PF4 exists as a tetramer in the α-granules of platelets and is secreted in high concentrations when platelets are activated. PF4 tetramer binds avidly to glycosaminoglycans (GAGs), but only a few high affinity protein receptors have been identified to date. The interaction of PF4 with GAGs is central to the pathogenesis of heparin induced thrombocytopenia as well as thrombosis.

Rauova, "Ultralarge Complexes of PF4 and heparin are central to the pathogenesis of heparin-induced thrombocytopenia", Blood, 105(1):131 (January 1, 2005), found that heparin:PF4 complexes exist in a dynamic equilibrium and that ultra large complexes (ULCs) are subject to dissociation under certain conditions.

Reilly, "Prothrombotic factors enhance heparin-induced thrombocytopenia and thrombosis in vivo in a mouse model", J. Thrombosis and Haemostatis, 4:2687-2694 (2006) (Reilly I) and Reilly, "Heparin-induced thrombocytopenia/thrombosis in a transgenic mouse model requires human platelet factor 4 and platelet activation through FcγRIIA", Blood, 98(8):2442-2447 (October 15, 2001) (Reilly II), discussed that the clinical manifestations of HITT are caused by antibodies that recognize a complex composed of heparin and PF4. The transgenic mouse model of HITT conclusively demonstrated that heparin, PF4, antiheparin/PF4 antibody, and platelet FcγRIIa are necessary and sufficient to recapitulate the salient features of HITT in mice.

While HIT and HITT remain a major problem associated with heparin treatment, there is an absence of a robust clinical algorithm to include or to exclude their diagnoses. Furthermore the hazard associated with continuing heparin in a patient with HIT; the risk of hemorrhage in a patient with post-operative thrombocytopenia erroneously diagnosed with HIT when given a direct thrombin inhibitor; the high false positive rate of currently formulated ELISAs; and limitations in current functional assays, all converge to highlight the need for a new, rapid, reliable, portable assay to measure antibodies likely to cause disease amidst a far larger number that do not activate cells and have a low probability of being pathogenic.

### SUMMARY OF THE INVENTION

In one aspect, the invention provides a method for identifying antibodies or fragments thereof that activate heparin-induced thrombocytopenia (HIT). The method includes contacting a hematopoietic cell or cell line that comprises, in operative association, the platelet receptor FcγRIIA under the control of a suitable promoter, and a reporter construct comprising a reporter gene under the control of a promoter and transcription factor, which transcription factor is regulated downstream of the signaling cascade of activated FcγRIIA, with a test sample from a mammalian subject; a platelet factor 4 (PF4), a wild-type or variant of PF4 or a fragment thereof; and heparin or a heparin-like molecule or a heparnoid molecule. The level of reporter gene expression is then detected or measured.

In another aspect, a method for screening molecules and compounds is provided. The method includes culturing a hematopoietic cell or cell line that comprises, in operative association, the platelet receptor FcγRIIA under the control of a suitable promoter and a reporter construct comprising a reporter gene under the control of a promoter and transcription factor, which is regulated downstream of the signaling cascade of activated FcγRIIA, with a test molecule, a platelet factor 4 (PF4), a wild-type or variant of PF4 or a fragment thereof; a heparin or a heparin-like molecule or a heparnoid molecule; and an FcγRIIA-activating antibody or fragment thereof. The level of reporter gene expression is then detected or measured.

In yet another aspect, a stable hematopoietic cell line is provided. The cell line comprises, in operative association, the platelet receptor FcγRIIA under the control of a suitable promoter and a reporter construct comprising a reporter gene under the control of a promoter and transcription factor, which is regulated downstream of the signaling cascade of activated FcγRIIA.

In another aspect, a method of assessing the risk of a mammalian subject for developing HIT is provided. The method comprises screening the subject's blood sample for the presence or absence of antibodies that cause HIT by utilizing one or more of the novel methods provided herein.

In yet another aspect, a method for monitoring a patient receiving heparin for the development of antibodies that cause HIT is provided. The method comprises screening the subject's blood sample at intervals before, during and after heparin therapy for the presence of antibodies that cause HIT by utilizing one or more of the novel methods provided herein.

In another aspect, an assay kit is provided. The assay kit comprises one or more of the following: a hematopoietic cell or cell line that comprises, in operative association, the platelet receptor FcγRIIA under the control of a suitable promoter and a reporter construct comprising a reporter gene under the control of a promoter and transcription factor, which is regulated downstream of the signaling cascade of activated FcγRIIA; suitable aliquot of platelet factor 4 (PF4), wild-type or variant of PF4 or a fragment thereof, or a panel of PF4s; a suitable aliquot of heparin or a heparin-like molecule or a heparnoid molecule or a panel of anionic anticoagulants; reagents for the detection of reporter gene expression; a positive control reference; a negative control reference; and suitable collection apparatus for a test sample from a mammalian subject or a test molecule.

Other aspects and advantages of the invention will be readily apparent from the following detailed description of the invention.

### BRIEF DESCRIPTION OF THE DRAWINGS

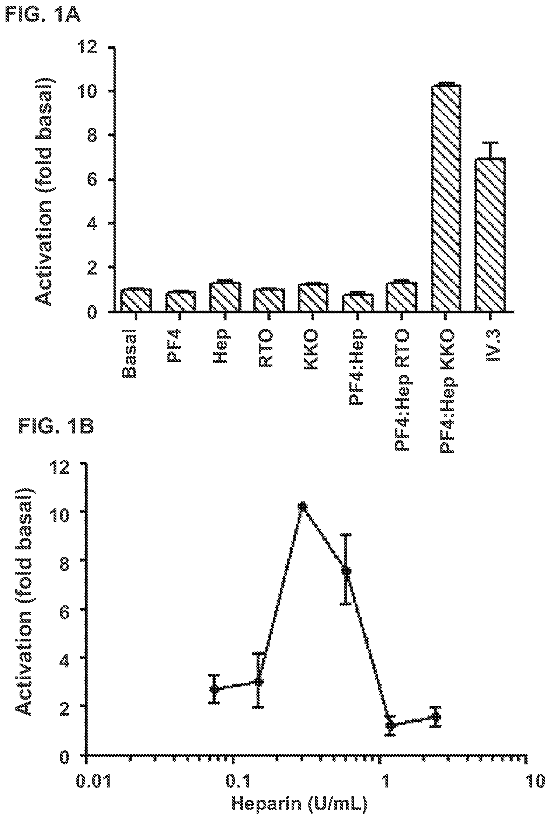

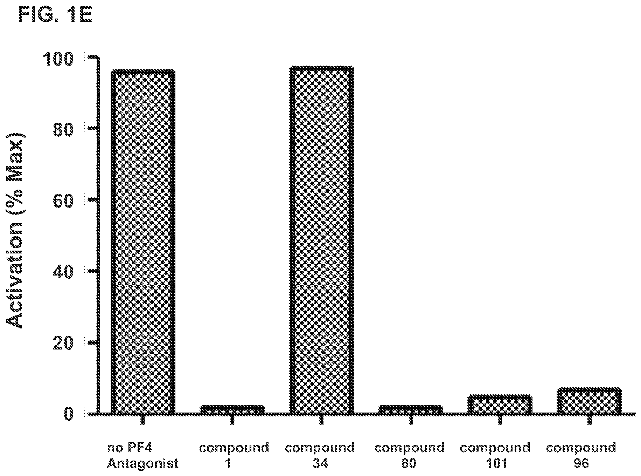

Figs. 1A-1F demonstrate FcγRIIA-mediated activation of cells and inhibition by PF4 antagonists: (A) Activation of DT 40 cells transfected with FcγRIIA and a Luc reporter. Basal condition is a buffer only control; Hep is heparin; IV.3 is the anti-FcγRIIA monoclonal antibody in the presence of anti-IgG antibody; RTO is an anti-P4 antibody; KKO is an anti-human PF4-heparin complex antibody. (B) Dose-response of DT40 activation by heparin in the presence of constant amounts of PF4. (C) Dose-response of antagonists for inhibition of DT40 activation. Data are the mean ± SEM of at least three independent experiments performed in triplicate. Curves in panel C represent fit of data to the indirect Hill equation. Closed circle data points are compound 1; inverted solid triangle data points are compound 17; open triangle data points are compound 80; open circle data points are compound 101; open diamond data points are compound 35. (D) Inhibition of DT40 activation by plasma obtained from three HIT patients. Both antagonists completely inhibited activation by KKO and patient samples at a concentration of 500 µM. Data represent the mean ± SEM of at least two independent experiments performed in triplicate. The middle bars with squares represent 100 µM compound 1. The right side bars with bricks represent 100 µM compound 101. (E) Activation of platelets as measured by release of ¹⁴C-5HT. Data are representative of at least two experiments. Antagonists were present at a concentration of 2.5 mM. (F) As in panel E except that activation was by plasma from patients with HIT and several antagonist concentrations were measured. Open circle data points represent compound 1. Open square data points represent compound 101.

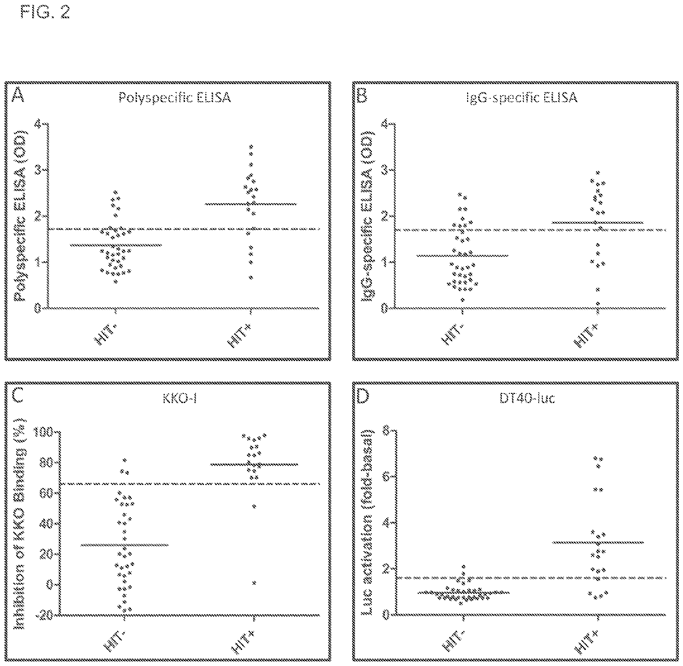

Figs. 2A-2D represent results from HIT-negative and HIT-positive subjects for the polyspecific ELISA, IgG-specific ELISA, KKO-I, and DT40-luc (panels A-D, respectively). Solid horizontal lines represent mean values. Dashed horizontal lines represent the cut-off associated with the most northwest point on the receiver operating characteristic curve (i.e. the cut-off at which sensitivity and specificity are optimized) for each assay.

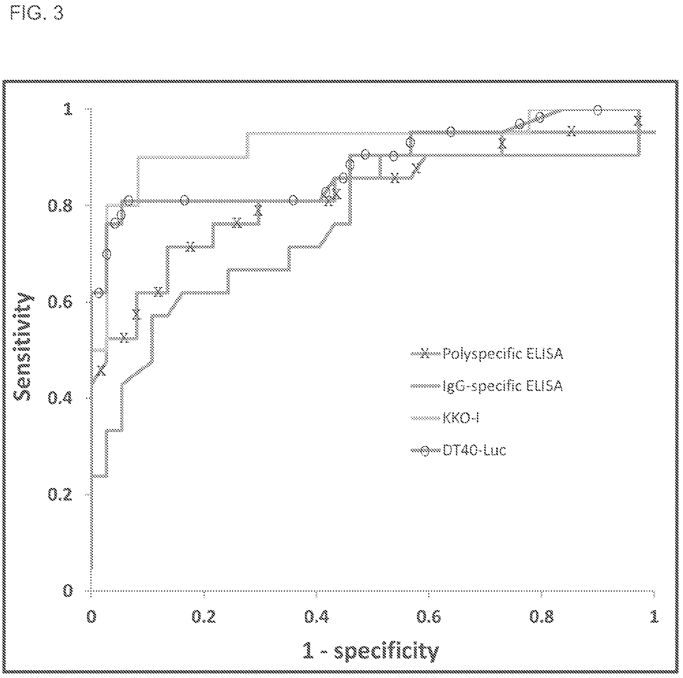

Fig. 3 shows ROC curves for the polyspecific ELISA, IgG-specific ELISA, KKO-I, and DT40-luc. The AUCs for these assays were 0.82, 0.76, 0.93, and 0.89, respectively. The AUC for KKO-I was significantly greater than the AUC for the polyspecific (p=0.020) and IgG-specific ELISA (p=0.0044). The AUC for DT40-luc was significantly greater than the AUC for the IgG-specific (p=0.046), but not the polyspecific ELISA (p=0.28).

### DETAILED DESCRIPTION OF THE INVENTION

A robust and reproducible new assay is provided to determine whether HIT-activating antibodies are present or absent in a sample. In addition, an assay is provided to measure inhibition of FcγRIIA-mediated cell activation caused by antibodies from patients with heparin-induced thrombocytopenia (HIT) in the presence of PF4 and heparin for the purposes of assessing the effect of PF4 antagonists.

HIT antibodies bound to PF4 on the surface of platelets initiate cellular activation by engaging FcγRIIA (Kelton et al, 1988 Blood, 72(3):925-930; Reilly et al, 2001, Blood, 98(8):2442-2447). However, few laboratories have the capacity to reliably work with platelets and provide clinically useful information in a clinically relevant timeframe.

The assay described herein has several advantages over functional assays currently used to assess HIT antibodies, i.e., the serotonin assay and the heparin-induced platelet activation assay. First, few laboratories have sufficient experience working with platelets and sufficient volume to maintain a functioning laboratory capable of providing clinically validated results to clinicians in real-time. In contrast, many labs have the capability of growing cells in culture. Second, many laboratories are prohibited from using the radioactivity required for the serotonin release assay. Third, inter-laboratory criteria for positive results and inter-laboratory agreement on test outcomes using the serotonin and platelet assays are poor. This is in part because platelets are both readily activated and inactivated during phlebotomy, washing and test performance. Fourth, the read out of platelet assays, aggregation or secretion, is a threshold rather than a linear response to activation and varies among donors.

In contrast, the assay provided herein involves transfecting the FcγRIIA receptor and an NFAT-Luc reporter using plasmids pEF6-FcγRIIA and pEF6-NFAT-Luc-luciferase reporter under control of the IL-2 promoter into mouse DT40 cells. The new assay provides an invariant source of cell type and a linear read-out that can be compared with other serologic or clinical outcomes, which is likely to be highly reproducible across laboratories and does not involve the use of radioactivity or fresh human platelets. Furthermore, the cell lines provided herein improve reliability for clinical purposes, and facilitate research across laboratories around the world.

Here, the inventors have shown that these cells are activated by the HIT-like monoclonal antibody KKO, as well as by antibodies from several patients with HIT, when PF4 and heparin are present and that activation is via FcγRIIA. Activation of DT40 cells follows the expected bell-shaped dose-response curve of heparin that characterizes the binding of HIT antibody to cells, formation of ultralarge PF4-heparin complexes in solution and platelet activation. This assay is thereby useful in measuring the inhibitory capacity of PF4 antagonists (and PF4 antagonist candidates) on FcγRIIA-dependent cellular activation.

### I. Definitions

The term "ULC", as used herein, refers to ultralarge complexes of heparin and PF4, which are the most pathogenic complexes of these components. Heparin:PF4 complexes smaller than 600 kDa are typically referred to as small complexes (SC). PF4:heparin ULCs are more pathogenic than heparin:PF4 SCs. Heparin:PF4 ULCs are better recognized by HITT antibodies and lead to more platelet activation in the presence of these antibodies. In one embodiment, ULCs are 600 kDa or larger. In another embodiment, ULCs are 670 kDa or larger. It has been determined that compounds which prevent formation of or disrupt the PF4 tetramer or ULC containing the same, *i*.*e*., antagonists, are useful in treating and/or preventing diseases related to the same. Such conditions include HITT since heparin:PF4 complexes are central to the pathophysiology of HITT as the targets of the pathogenic antibodies.

The term "PF4" as used herein refers to platelet factor 4 which is a 70 amino acid, lysine-rich, 7.8 kDa platelet-specific protein that belongs to the CXC (or beta) chemokine subfamily, in which the first two of the four conserved cysteine residues are separated by one amino acid residue. The PF4 may be derived from any species that natively expresses the protein. In one embodiment, PF4 is naturally occurring, *i*.*e*., wild-type. In another embodiment, PF4 may be synthesized by recombinant or chemical methods. In another embodiment, PF4 also refers to variants or mutants thereof in which one or more of the amino acids is replaced with a different amino acid. Examples of PF4 mutations are described in International Patent Publication No. WO 02/006300. In one example, the mutated PF4 contains Arg28 and/or Glu50 mutations. In addition, fragments of PF4 including functional fragments are also encompassed by the term. In one embodiment, the PF4 is human PF4.

The term "antagonist" as used herein refers to any chemical compounds which are capable of binding to a PF4 tetramer, dimer or monomer. By doing so, the compound is thereby capable of preventing formation of or disrupting ULCs formed between the PF4 tetramer. Specifically, ULCs formed between glycosaminoglycans (GAG) and PF4 tetramers are responsible for the onset or development of individuals at risk for certain diseases or medical conditions. Therefore, desirable compounds are those which disrupt or prevent the formation of the PF4 tetramer and ULCs formed therewith. By doing so, the antagonist compound prevents formation of, or disrupts, ULCs.

The term "heparin" as used herein refers to a heparin or a preparation derived there from, and thus includes unfractionated heparin, low molecular weight heparin (LMWH), ultra-low molecular weight heparin (ULMWH) and the like. In addition heparin-like molecules and heparinoid molecules are encompassed by the term. Heparin may be derived from any mammalian animal that produces the molecule. In one embodiment, the heparin is derived from a pig. In another embodiment, the heparin is derived from a cow. In another embodiment, heparin is derived from a human. In another embodiment, the heparin is synthetic.

The term "antibody", as used herein, refers to any naturally occurring or synthetic antibody or fragment thereof. Antibodies used in the methods of the invention include monoclonal antibodies, polyclonal antibodies, antibody fragments (e.g., Fab, and F(ab')₂) and recombinantly produced binding partners. Polyclonal antibodies may be readily generated by one of ordinary skill in the art from a variety of warm-blooded animals such as horses, cows, various fowl, rabbits, mice, or rats. Monoclonal antibodies may also be readily generated using conventional techniques.

### II. Cells and Cell Lines

In one aspect, the invention provides a hematopoietic cell or cell line. The cell or cell line comprises, in operative association, the platelet receptor FcγRIIA under the control of a suitable promoter and a reporter construct comprising a reporter gene under the control of a promoter and transcription factor, which is regulated downstream of the signaling cascade of activated FcγRIIA.

As used herein, the term "hematopoietic cell" or "cell line" refers to any cell, cell type or cell line derived from bone marrow, and T cells. In one embodiment, the hematopoietic cell or cell line may be myeloid cells including platelets, neutrophils, monocytes and macrophages, eosinophils, basophils, or mast cells or lymphoid cells including B cells, T cells, or NK-cells. Further, the hematopoietic cell or cell line may be derived from any mammalian animal or bird. In one embodiment, the cell line is derived from a human, chicken, mouse, pig, non-human primate, dog, cat, rat, hamster, bovine, ovine or bird. In one embodiment, the hematopoietic cell is a B lineage cell or cell line. In another embodiment, the hematopoietic cell is a T lineage cell or cell line. In yet another embodiment, the hematopoietic cell is a chicken DT-40 B cell. In another embodiment, the hematopoietic cell is a human Jurkat T cell line. In another embodiment, the hematopoietic cell is a rat RBL-2H3 mast cells.

Antibodies to the PF4/heparin complex have been shown to activate human platelets *in vitro* via the platelet Fc receptor (FcR) for immunoglobulin (Ig)-G, FcγRIIA. FcγRII (also called CD32) is a 40,000 Da receptor for the Fc region of Immunoglobulin G (IgG). CD32 is encoded by three genes, A, B, and C, and is expressed as 6 protein isoforms, IIa1, IIa2, IIb1, IIb2, IIb3 and IIc. The IIa isoform is expressed on platelets, granulocytes and weakly on B cells. All isoforms are expressed on monocytes/macrophages. The sequence of FcγRIIA is readily available, e.g., see I.M.A.G.E. gene symbol FCGR2A, Unigene ID: Hs.352642. As used herein, the term "IV.3" refers to the mouse monoclonal antibody IV.3 which binds to CD32 (FcγRII) on human cells. In another embodiment, IV.3 refers to the IV.3 antibody in combination with an anti-Fc antibody to activate FcγRIIA bound by IV.3. Antibody IV.3 is commercially available or may be readily produced using methods well known in the art. The FcγRIIA sequence may be derived from any mammalian animal which natively expresses the protein including, but not limited to, human, mouse, bovine, porcine, rat, or non-human primate. In one embodiment, the FcγRIIA sequence is a human sequence.

The platelet receptor FcγRIIA and the transcription factor/reporter construct utilized in the assay are regulated by suitable promoters. A number of expression control sequences, including promoters which are native, constitutive, inducible and/or tissue-specific, are known in the art and may be utilized. Promoters which may be used include, but are not limited to, the long terminal repeat, the SV40 early promoter region, the CMV promoter, the M-MuLV 5'-terminal repeat, the promoter contained in the 3' long terminal repeat of Rous sarcoma virus, the herpes thymidine kinase promoter, the regulatory sequences of the metallothionine gene; prokaryotic expression vectors such as the β-lactamase promoter, or the tac promoter; or promoter elements from yeast or other fungi such as the Gal 4 promoter, the ADH (alcohol dehydrogenase) promoter, PGK (phosphoglycerol kinase) promoter, alkaline phosphatase promoter. In one embodiment, the promoter may be the EF1, CMV, FerL, or IL-2 promoter. In another embodiment, the FcγRIIA coding sequence is under the control of the human EF-1α promoter.

In one embodiment, the transcription factor/reporter construct comprises a reporter gene under the control of a composite promoter comprising the nuclear factor of activated T-cells (NFAT)-AP-1 sites from the IL-2 promoter. NFAT is a general name applied to a family of transcription factors shown to be important in immune response. The NFAT reporter contains three copies of a composite NFAT-activator protein-1 (AP-1) element from the human interleukin-2 (IL-2) gene promoter, and is maximally activated by combined Ca²⁺ elevation and RAS/mitogen-activated protein kinase (MAPK) signaling, which activate NFAT and AP-1, respectively. Both Ca²⁺ and map kinase (MAPK) are generated by the signaling cascade of activated FcγRIIA. NFAT-reporter assays are well known in the art and are commercially available, from, e.g., SABioscience and Addgene. See, Tomlinson et al, J Thromb Haemost 2007; 5: 2274-83. In one embodiment, NFAT is selected from NFATc1, NFATc2, NFATc3, and NFATc4, all of which are regulated by Ca²⁺.

The reporter gene sequence may be selected based on the desired assay format. Such reporter sequences include, without limitation, DNA sequences encoding β-lactamase, β-galactosidase (LacZ), alkaline phosphatase, thymidine kinase, green fluorescent protein (GFP), red fluorescent protein (RFP), chloramphenicol acetyltransferase (CAT), luciferase, membrane bound proteins including, for example, CD2, CD4, CD8, the influenza hemagglutinin protein, and others well known in the art, to which high affinity antibodies directed thereto exist or can be produced by conventional means, and fusion proteins comprising a membrane bound protein appropriately fused to an antigen tag domain from, among others, hemagglutinin or Myc. These coding sequences, when associated with regulatory elements which drive their expression, provide signals detectable by conventional means, including enzymatic, radiographic, colorimetric, fluorescence or other spectrographic assays, fluorescent activating cell sorting assays and immunological assays, including enzyme linked immunosorbent assay (ELISA), radioimmunoassay (RIA) and immunohistochemistry. For example, where the marker sequence is the LacZ gene, the presence of the vector carrying the signal is detected by assays for beta-galactosidase activity. Where the transgene is GFP or luciferase, the vector carrying the signal may be measured visually by color or light production in a luminometer. In one embodiment, the reporter gene is luciferase.

The hematopoietic cells of the invention may be prepared using conventional techniques which are well known in the art. See, e.g., Example 1 and Sambrook et al, Molecular Cloning: A laboratory manual, 3rd Ed., Cold Spring Harbor Press, 2001. In one embodiment, the hematopoietic cell is co-transfected with a first plasmid expressing FcγRIIA under the control of a suitable promoter, and a second plasmid carrying the reporter construct. In another embodiment, the hematopoietic cell or cell line is a transiently transfected cell containing the two plasmids. In another embodiment, hematopoietic cell or cell line is a stable cell line, i.e., the FcγRIIA construct and reporter construct have been integrated into the host cell's genome. Stable cell lines can readily be created by those of skill in the art in any desired hematopoietic cell line, as described above, using readily available protocols and techniques. See, e.g., Example 5 below and Teixeira et al, Journal of Biotechnology 88 (2001) 159-165 and Sambrook et al, cited above.

### III. Methods

### A. Identifying HIT-inducing Antibodies

In another aspect, the invention provides a method for identifying antibodies or fragments thereof that activate HIT. In one embodiment, the method comprises contacting a hematopoietic cell or cell line, discussed above, with a test sample from a mammalian subject, a platelet factor 4 (PF4), and heparin or a heparin-like molecule or a heparinoid molecule; and detecting or measuring the level of reporter gene expression. The hematopoietic cell or cell line comprises, in operative association, the platelet receptor FcγRIIA under the control of a suitable promoter, and a reporter construct comprising a reporter gene under the control of a promoter and transcription factor, which transcription factor is regulated by products downstream of the signaling cascade of activated FcγRIIA. In one embodiment, the cell or cell line is any of the cells or cell lines described above.

Without wishing to be bound by theory the inventors hypothesized that, in the presence of an HIT-activating antibody, the antibody and PF4-heparin ULC bind to, and activate FcγRIIA. The resulting signaling cascade generates products, i.e., Ca²⁺ and MAP kinase, which bind NFAT and AP-1, respectively, activating the promoter of the reporter construct, producing an increase in reporter gene expression. In the absence of an HIT-activating antibody, no increase in reporter gene expression is seen. Thus, in one embodiment, when the test sample contains an HIT-activating antibody or fragment, the antibody and PF4-heparin complexes in contact with the cell or cell line activate FcγRIIA and its signaling cascade resulting in increase in reporter gene expression. In another embodiment, when the test sample does not contain an HIT-activating antibody or fragment, FcγRIIA and its signaling cascade are not activated, and no increase in reporter gene expression occurs. Under this paradigm, the assay provides a method of diagnosing HIT in a subject.

In one embodiment, the subject providing the test sample is a mammalian subject. The mammalian subject may be a human or any domestic, laboratory or farm animal. In one embodiment, the term mammalian subject includes human, mouse, pig, horse, cat, non-human primate, dog, rat, hamster, goat, bovine or ovine. In a preferred embodiment, the mammalian subject is a human.

The test sample may be any biological sample derived from the test subject. In one embodiment, the sample is any biological sample which may contain antibodies or fragments thereof. In one embodiment, the test sample is blood, plasma, serum, cell eluate, or a recombinant protein. In another embodiment, the test sample is saliva, cerebral spinal fluid, pleural fluid, milk, lymph, sputum, semen, urine, stool, tears, needle aspirates, tissue, or tumor or organ section.

In one embodiment, the reporter gene expression in the cells containing the test sample or test molecule is compared to a reference. In one embodiment, the reference is a basal level of reporter expression generated by the cells in the absence of an HIT-activating antibody or in the presence of a negative control or antibody that does not activate FcγRIIA in the cells. In one embodiment, the negative control is the RTO (anti-P4) antibody. In another embodiment, the negative control is human plasma from a subject without HIT. In another embodiment, the negative control is a non-antigenic PF4 that cannot form ultra large complexes with heparin. In another embodiment the negative control is an anti-coagulant that does not form ultra large complexes with PF4. In one embodiment, a reporter gene expression level of about 1.2 fold of the basal level indicates a positive test or diagnosis of HIT in the subject. In one embodiment, a reporter gene expression level of about 1.3 fold of the basal level indicates a positive test or diagnosis of HIT in the subject. In one embodiment, a reporter gene expression level of about 1.4 fold of the basal level indicates a positive test or diagnosis of HIT in the subject. In one embodiment, a reporter gene expression level of about 1.5 fold of the basal level indicates a positive test or diagnosis of HIT in the subject. In one embodiment, a reporter gene expression level of about 1.6 fold of the basal level indicates a positive test or diagnosis of HIT in the subject. In one embodiment, a reporter gene expression level of about 1.7 fold of the basal level indicates a positive test or diagnosis of HIT in the subject. In one embodiment, a reporter gene expression level of about 1.8 fold of the basal level indicates a positive test or diagnosis of HIT in the subject. In one embodiment, a reporter gene expression level of about 1.9 fold of the basal level indicates a positive test or diagnosis of HIT in the subject. In one embodiment, a reporter gene expression level of about 2.0 fold of the basal level indicates a positive test or diagnosis of HIT in the subject. In one embodiment, a reporter gene expression level of about 2.1 fold of the basal level indicates a positive test or diagnosis of HIT in the subject. In one embodiment, a reporter gene expression level of about 2.2 fold of the basal level indicates a positive test or diagnosis of HIT in the subject. In one embodiment, a reporter gene expression level of about 2.3 fold of the basal level indicates a positive test or diagnosis of HIT in the subject. In one embodiment, a reporter gene expression level of about 2.4 fold of the basal level indicates a positive test or diagnosis of HIT in the subject. In one embodiment, a reporter gene expression level of about 2.5 fold of the basal level indicates a positive test or diagnosis of HIT in the subject. In one embodiment, a reporter gene expression level of about 2.6 fold of the basal level indicates a positive test or diagnosis of HIT in the subject. In one embodiment, a reporter gene expression level of about 2.7 fold of the basal level indicates a positive test or diagnosis of HIT in the subject. In one embodiment, a reporter gene expression level of about 2.8 fold of the basal level indicates a positive test or diagnosis of HIT in the subject. In one embodiment, a reporter gene expression level of about 2.9 fold of the basal level indicates a positive test or diagnosis of HIT in the subject. In one embodiment, a reporter gene expression level of about 3 fold or greater of the basal level indicates a positive test or diagnosis of HIT in the subject.

In another embodiment, the reference is a level of reporter expression generated in the presence of a positive control that activates FcγRIIA in the cells. In one embodiment, the positive control is the IV.3 antibody in combination with an anti-Fc antibody to activate FcγRIIA bound by IV.3. In another embodiment, the positive control is the KKO (anti-human PF4-heparin complex) antibody. In another embodiment, the positive control is plasma from a subject with known HIT. In yet another embodiment, the reference is a level of reporter expression generated in the absence of a test sample or test molecule. In another embodiment, the reporter gene expression is increased in a heparin- and PF4- dose dependent manner.

### B. Diagnostic Methods

In another aspect, here disclosed is a method to assess the risk of a mammalian subject for developing HIT or HITT. The method includes screening the subject's test sample for the presence or absence of antibodies that cause HIT using any of the methods described above.

The test sample may be any of those described above. In one embodiment, the test sample is blood. In another embodiment, the test sample is plasma. In another embodiment, the sample is serum.

In one embodiment, the method is performed after the patient has initiated with heparin administration. In one embodiment, the method is repeated over the course of treating a patient with heparin. In another embodiment, the method is performed prior to beginning treatment with heparin. In yet another embodiment, the method is performed prior to beginning treatment with heparin when the patient has been previously treated with heparin within the last 6 months. In yet another embodiment, the method is performed prior to beginning treatment with heparin when the patient has been previously treated with heparin within the last 3 months.

This method thereby permits detection of antibodies and correlates the same with HIT or HITT. In one embodiment, when HIT-inducing antibodies are detected in the test sample, the patient is at risk for developing HIT or HITT. In another embodiment, when HIT-inducing antibodies are detected in the test sample, the patient has HIT or HITT.

### C. Therapeutic Methods

In another aspect, here disclosed is a method for monitoring a patient receiving heparin for the development of antibodies that cause HIT. The method includes screening the subject's test sample at intervals before, during and after heparin therapy for the presence of antibodies that cause HIT, using the methods described above.

The test sample may be any of those described above. In one embodiment, the test sample is blood. In another embodiment, the test sample is plasma. In another embodiment, the sample is serum.

In one embodiment, when HIT-inducing antibodies are detected in the sample, heparin therapy is discontinued.

### D. Screening Methods

In another aspect, a method for screening molecules and compounds is provided. In one embodiment, the method comprises culturing a hematopoietic cell or cell line with a test molecule; a platelet factor 4 (PF4), a wild-type or variant of PF4 or a fragment thereof; heparin or a heparin-like molecule or a heparnoid molecule; and an FcγRIIA-activating antibody or fragment thereof. The method further comprises detecting or measuring the level of reporter gene expression. In one embodiment, the cell or cell line is selected from those described above. In another embodiment, the cell or cell line contains, in operative association, the platelet receptor FcγRIIA under the control of a suitable promoter and a reporter construct comprising a reporter gene under the control of a promoter and transcription factor, which is regulated downstream of the signaling cascade of activated FcγRIIA.

Any of the hematopoietic cells or cell lines described above may be used in the screening methods of the invention. For conciseness, each possible embodiment of each component is not repeated here. However, it is intended that each of the embodiments of the assay components shall have the same scope as the embodiments described above.

Without wishing to be bound by theory, the inventors hypothesize that, in the presence of a PF4 antagonist compound, the HIT-activating antibody cannot bind to the PF4-heparin ULC (or binding is reduced), and thus cannot activate FcγRIIA. Thus, in the presence of a PF4 antagonist compound, no increase in reporter gene expression is seen. In the absence of a PF4 antagonist compound, the HIT-activating antibody binds the ULC, and activates FcγRIIA. The resulting signaling cascade generates products, i.e., Ca²⁺ and MAP kinase, which bind NFAT and AP-1, respectively, activating the promoter of the reporter construct, producing an increase in reporter gene expression.

Thus, in one embodiment, when the test molecule is a PF4 antagonist which can disrupt the formation of ultra large particles comprising PF4 and heparin, FcγRIIA and its signaling cascade are not activated, and no increase in reporter gene expression occurs.

In another embodiment, when the test molecule is a non-activating FcγRIIA antagonist or non-activating FcγRIIA antibody, FcγRIIA and its signaling cascade are not activated, and no increase in reporter gene expression occurs.

In yet another embodiment, when the test molecule is unable to bind FcγRIIA or PF4 so as to block activation or the formation of ultra-large complexes, the FcγRIIA-activating antibody and PF4-heparin complexes in contact with the cell or cell line activate FcγRIIA and its signaling cascade, thereby generating downstream products that activate the transcription factor and promoter of the reporter construct, resulting in an increase in reporter gene expression.

In one embodiment, the test molecule is a compound. In another embodiment, the test molecule is a chemical entity. In another embodiment, the test molecule is a small molecule. In another embodiment, the test molecule is an antibody. In another embodiment, the test molecule is a peptide. In another embodiment, the test molecule is a protein. In another embodiment, the test molecule is a carbohydrate. In another embodiment, the test molecule is a nucleic acid sequence.

In another embodiment, the expression of the reporter gene is compared to a reference. In one embodiment, the reference is a basal level of reporter expression generated by the cells in the absence of an HIT-activating antibody or in the presence of a negative control that does not activate FcγRIIA in the cells. In this embodiment, a test result which correlates with a negative (or decreased) result in the presence of a compound, combined with a positive result in the absence of that compound indicates a compound which is a PF4 antagonist. The negative control can be any of those described above. In one embodiment, the negative control is human plasma from a subject without HIT. In another embodiment, the negative control is a non-antigenic PF4 that cannot form ultra large complexes with heparin. In another embodiment, the negative control is an anti-coagulant that does not form ultra large complexes with PF4. In another embodiment, the reference is a level of reporter expression generated in the presence of a positive control that activates FcγRIIA in the cells. In this embodiment, a test result which correlates with a positive control indicates a compound which is not a PF4 antagonist. In one embodiment, the positive control is the IV.3 antibody in combination with an anti-Fc antibody to activate FcγRIIA bound by IV.3. In another embodiment, the positive control is the KKO (anti-human PF4-heparin complex) antibody. In yet another embodiment, the reference is a level of reporter expression generated in the absence of a test sample or test molecule. In another embodiment, the reporter gene expression is increased in a heparin- and PF4- dose dependent manner.

### IV. Kits

In another aspect, assay kits are provided. In one embodiment, the assay kit comprises one or more of: a hematopoietic cell or cell line of the invention; a suitable aliquot of platelet factor 4 (PF4), wild-type or variant of PF4 or a fragment thereof, or a panel of PF4s; a suitable aliquot of heparin or a heparin-like molecule or a heparnoid molecule or a panel of anionic anticoagulants; reagents for the detection of reporter gene expression; a positive control reference; a negative control reference; and suitable collection apparatus for a test sample from a mammalian subject or a test molecule. As described above, the hematopoietic cell or cell line comprises, in operative association, the platelet receptor FcγRIIA under the control of a suitable promoter and a reporter construct comprising a reporter gene under the control of a promoter and transcription factor, which is regulated downstream of the signaling cascade of activated FcγRIIA.

In one embodiment, the assay kit comprises any of the cells or cell lines described above. For conciseness, each and every embodiment of each component is not repeated here. However, it is intended that each of the embodiments of the assay components shall have the same scope as the embodiments described above.

Suitably, the kit contains packaging or a container with each of the above-described components. In one embodiment, the kit contains instructions on how to perform the assay and optionally, additional materials for performing such assays including, *e.g.*, reagents, well plates, containers, markers or labels, and the like.

The compositions of these kits also may be provided in dried or lyophilized forms. When reagents or components are provided as a dried form, reconstitution generally is by the addition of a suitable solvent. It is envisioned that the solvent also may be provided in another packaging means.

The kits here disclosed also will typically include a means for containing the vials in close confinement for commercial sale such as, e.g., injection or blow-molded plastic containers into which the desired vials are retained. Other instrumentation includes devices that permit the reading or monitoring of reactions *in vitro.*

The following examples are illustrative of methods using the antagonistic compounds described herein. It will be readily understood by one of skill in the art that the specific conditions described herein for producing these compounds can be varied without departing from the scope of the present invention. It will be further understood that other compounds encompassed by the structures described herein, as well as other salts, hydrates, and/or prodrugs thereof, are within the scope of the invention.

### EXAMPLES

Compounds which are believed to be useful in preventing and treating HIT and HITT are described in a related application entitled "SMALL MOLECULE ANTAGONISTS OF PF4 CONTAINING ULTRA LARGE COMPLEXES" which is filed concurrently herewith. Several compounds from that application were used as a model in testing the novel assays and cell lines of the present invention and are referred to by their compound numbers used in that application (e.g., compounds 1, 34, 80, 96 and 101 listed in Table 1 below).

**Table 1: PF4 Antagonist Compounds**

| | |
|---|---|
| 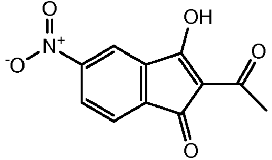 | 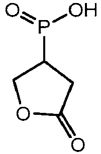 |
| 1 | 34 |
| 2-acetyl-3-hydroxy-5-nitro-inden-1-one | (5-oxotetrahydrofuran-3-yl)phosphinic acid |
| 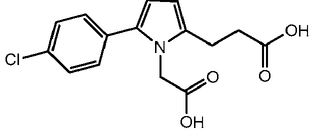 | |
| 80 | |
| 3-[1-(carboxymethyl}-5-(4-chlorophenyl)-pyrrol-2-yl]propanoic acid | |
| 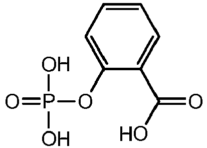 | 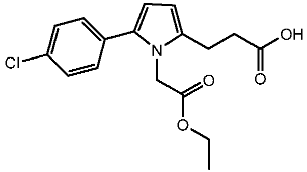 |
| 96 | 101 |
| 2-phosphonooxybenzoic acid | 3-[5-(4-chlorophenyl)-1-(2-ethoxy-2-oxyethyl)-1H-pyrrol-2-yl] propanoic acid |

Unfractionated heparin was an injectable sodium salt from porcine intestinal mucosa (Sagent Pharmaceuticals, Schaumberg, IL). Murine monoclonal antibodies KKO (anti-human PF4-heparin complex), RTO (anti-human PF4), and IV.3 (FcyRIIA-blocking antibody) may be prepared according to Arepally, "Characterization of a murine monoclonal antibody that mimics heparin-induced thrombocytopenia antibodies", Blood, 95(5):1533-1540 (2000). Human plasma samples were obtained from patients with a high clinical suspicion for HIT (see, "Cuker, "The HIT expert probability (HEP) score: a novel pre-test probability model for heparin-induced thrombocytopenia based on broad expert opinion", J. Thromb. Haemost., 8(12):2642-2650 (2010), and who had positive heparin/PF4 ELISA (GTI X-HAT45; Waukesha, WI) and serotonin release assay results. Dose response data were analyzed using the indirect Hill relationship using GraphPad® Prism® software (version 5.03, sigmoidal dose-response, variable slope model).

### EXAMPLE 1: Preparation of B cells containing NFAT-Luc and FcγRIIA plasmids.

B cells used in the novel assays of the invention were prepared as follows. NFAT-Luc was kindly provided by Prof. Arthur Weiss (Shapiro, "c-rel Regulation of IL-2 gene expression may be mediated through activation of AP-1", J. Exp. Med., 184(5):1663-1669 (1996). The NFAT reporter contains three copies of a composite NFAT-activator protein-1 (AP-1) element from the human interleukin-2 (IL-2) gene promoter, and is activated by binding of NFAT (nuclear factor of activated T-cells). pEF6-FcγRIIA, which contains the human FcγRIIA coding sequence under the control of the human EF-1a promoter, was constructed by cloning a human FcγRIIA IMAGE clone into the multiple cloning site of pEF6c (Invitrogen).

DT40 cells (chicken B cells) were cultured in RPMI supplemented with 10% fetal bovine serum, 1% chicken serum (Gibco), 50 µM 13-mercaptoethanol, 2 mM GlutaMAX (Gibco), 100 U/mL penicillin and 100 U/mL streptomycin at 37°C under 5% CO₂. Cells were co-transfected with 5 µg pEF6-FcγRIIA and 20 µg pEF6-NFAT-Luc by electroporation. Positive transformants were selected using blastidicin.

### EXAMPLE 2: HIT antibodies bound to ULC on platelets initiate activation by engaging FcγRIIA.

To validate that the transfected cells are useful in measuring inhibition of FcγRIIA mediated activation by HIT antibodies in the presence of PF4 and heparin the following experiments were performed.

On day following transfection, cells were pelleted by centrifugation and resuspended at 2 x 10⁶ cells/mL in fresh medium without serum. Cell aliquots (50 µL) were placed into each well of a 96 well plate. To establish basal expression, 50 µL media was added. Further, the cells were also incubated independently with the following: PF4, heparin, RTO antibody, KKO antibody, ULCs alone, ULCs and RTO; ULCs and KKO and IV.3 antibody. Monoclonal antibody IV.3 (an FcγRIIA-blocking antibody) was used in combination with an anti-Fc antibody to activate FcγRIIA bound by IV.3 as a positive control for FcγRIIA signaling. 8 µg/mL of IV.3 was added for 15 minutes at 37°C under 5% CO₂, followed by 50 µL of sheep anti-mouse-lgG secondary antibody at 15 µg/mL to crosslink and activate these receptors. As a positive control for an intact intracellular signaling pathway, phorbol myristate acetate (PMA; 25 ng/mL) with ionomycin (1µM) was added (not shown).

The data show that the cells are activated by the anti-FcγRIIA monoclonal antibody IV.3 in combination with an anti-Fc antibody to activate FcγRIIA bound by IV.3, as well as by the HIT-like monoclonal antibody KKO in the presence of ULC (Figure 1A). However, RTO, an isotype control that binds avidly to PF4 but does not mimic the pathological properties of HIT, failed to activate these cells. Importantly, KKO/ULC did not activate cells lacking FcγRIIA (transfected with pEF6-NFAT-Luc alone; data not shown) demonstrating that activation in this system is FcγRIIA dependent. Furthermore, addition of PF4, heparin, KKO, or RTO alone did not activate the FcγRIIA-expressing cells, demonstrating the relevance of this assay to measuring the functional activity of HIT antibodies (Fig. 1A).

It was next examined whether FcγRIIA activation was heparin-dependent. To do so, activation by KKO was measured using a constant amount of PF4 and increasing amounts of heparin. As seen in Figure 1B, the previously described bell-shaped dose-dependence of heparin concentration on platelet activation was observed. Taken together, the data support the use of the DT40 cell activation system as an assay of cellular activation via FcγRIIA dependent on PF4, heparin and HIT antibody and therefore as a means to assess the effect of PF4 antagonists discussed herein.

See, Kelton, "Heparin-induced thrombocytopenia: Laboratory studies", Blood, 72(3):925-930 (1988) and Reilly, "Heparin-induced thrombocytopenia/thrombosis in a transgenic mouse model requires human platelet factor 4 and platelet activation through FcγRIIA", 98(8):2442-2447 (2001).

### EXAMPLE 3: Inhibition of FcγRIIA-dependent cellular activation by PF4 antagonists

We then used this assay to measure the inhibitory capacity of the PF4 antagonist compounds described in the concurrently filed related application referenced above. PF4 (10 µg/mL) and various concentrations of potential antagonists were co-incubated for 60 min at 37°C under 5% CO₂ followed by the addition of heparin (0.3 U/mL) for 15 min at 37°C in 5% CO₂. KKO (20 µg/mL) or plasma from patients with serotonin release assay confirmed HIT (1:800 dilution) was then added. 50 µL of each mixture was added to the 96 well plate containing 50 µL of cells at a concentration of 2 x 10⁶ cells/mL in fresh medium without serum. Plates were incubated for 6 hours at 37°C under 5% CO₂, and then frozen at -80°C. To measure activation, cells were thawed and lysed with 5X Passive Lysis Buffer (Promega) for 15 minutes. Luciferase activity was measured on a Berthold MultiLumat LB 9506 Luminometer (10 sec readings) using Promega's Luciferase Assay Reagent following the manufacturer's instructions. In other experiments, PF4 antagonists discussed herein were incubated with PF4 for 5 minutes with the same results.

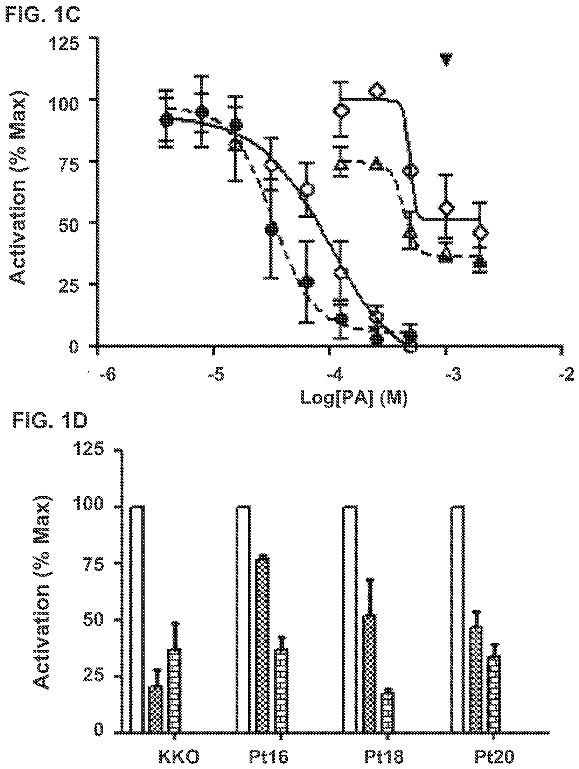

Compounds 1 and 101 were the most potent inhibitors of cellular activation (Table 2 and Fig. 1C), causing complete inhibition at the highest doses tested. Compounds 80 and 96 were less potent and, as expected, compound 34 was not inhibitory. Importantly, compounds 1 and 101 both inhibited activation by human HIT plasma with a similar potency as activation by KKO (Figure 1D).

**Table 2**

| **PF4 Antagonist** | **IC₅₀ (µM)** |
|---|---|
| 1 | 33 |
| 34 | - |
| 80 | 440 |
| 101 | 100 |
| 96 | 500 |

### EXAMPLE 4: Serotonin Release Assay

To confirm the results of the novel assay, an independent, orthogonal assessment of activity was performed using a serotonin release assay. Serotonin release assays are described in Hirschman, "The use of platelet serotonin release as a sensitive method for detecting anti-platelet antibodies and a plasma anti-platelet factor in patients with idiopathic thrombocytopenic purpura", Br. J. Haematol., 24(6):793-802 (1973), and Rauova and Cines, "Heparin-associated thrombocytopenia", N. Engl. J. Med., 303:788-795 (1980).

Briefly, platelet rich plasma (PRP) from healthy donors was incubated with 0.5 µL ¹⁴C-5-hydroxytryptamine creatinine sulfate (GE Life Sciences, Piscataway, NJ) per milliliter of PRP for 20 minutes at 37°C to produce ¹⁴C-labeled platelets. Serotonin uptake was inhibited by adding 1 mmole/mL imipramine (Sigma-Aldrich, St. Louis, MO) to the PRP.

The radiolabeled platelets were mixed with KKO (170 µg/mL) or with known platelet-activating HIT plasma in the absence (buffer control) or presence of PF4 antagonist compounds 1, 34, 80, 101, and 96. Assays were performed in the presence of heparin (1.0 U/mL) and in the absence of heparin (background). Negative controls without antibody were studied in parallel. Data are expressed as % maximal release of radioactivity with release by the positive control plasma and 1.0 U/mL heparin defined as 100%.

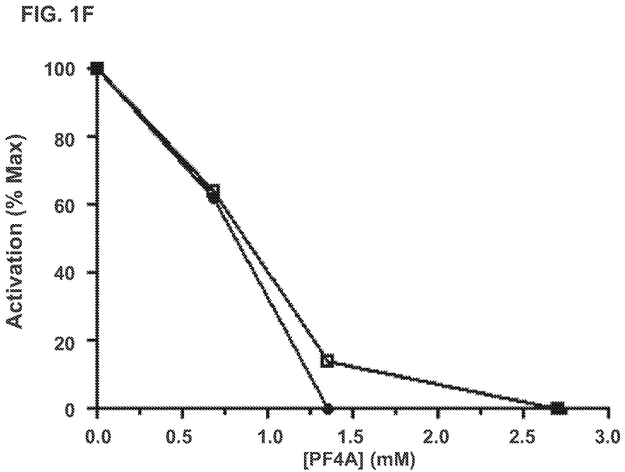

Compounds 1, 80, 101 and 96 inhibited platelet activation by KKO as measured by the serotonin release assay (Figure IE). The rank order of potency for inhibition of cellular activation by these compounds paralleled their ability to inhibit tetramer formation. Further, compounds 1 and 101 inhibited serotonin release from platelets activated by plasma from patients with HIT (Figure 1F).

### EXAMPLE 5: Stable Cell Line

Cells are transfected as in Example 1 and plated into 5 petri dishes. Cells are returned to the incubator for 24 hours at 37°C under 5% CO₂. The supernatant is harvested and stored at -20°C until ready to assay the CLuc activity. The medium is replaced and the cells are returned to the incubator for 24 hours. RPMI supplemented with 10% fetal bovine serum, 1% chicken serum (Gibco), 50 µM 13-mercaptoethanol, 2 mM GlutaMAX (Gibco), and 5µg/mL blastidicin is added to the plates and they are incubated for another 24 hours. Media is changed daily until the majority (∼99%) of cells are dead and colonies begin to appear (∼7-10 days). Media is replaced and the colonies are isolated and transferred to a 96-well plate. Medium is replaced for the isolated clones every 2-3 days until the cell density reaches ∼80%.

Colonies are incubated with KKO antibody at 20 µg/mL and checked for luciferase expression to verify positive clones. Positive clones are expanded to larger vessels, stepwise, until sufficient cell stock has been generated for freezing.

### EXAMPLE 6: Comparison with other methods of detecting HIT

The performance of the DT40-luc assay was compared with a KKO-I assay described in US Provisional Patent Application No. 61/640,960 and two commercially available immunoassays in samples from 58 patients with suspected HIT and circulating anti-PF4/heparin antibodies.

Patient samples consecutively referred to the University of Pennsylvania for laboratory assessment of HIT that tested positive [i.e. optical density (OD) ≥0.40] in a polyspecific PF4/heparin ELISA (Hologic Gen-Probe, San Diego, CA) were included. Citrated plasma samples from all patients were also tested using an IgG-specific PF4/heparin ELISA (Hologic Gen-Probe), an in-house SRA, and the investigational KKO-I and DT40-luc assays. The polyspecific and IgG-specific ELISAs were performed in accordance with the manufacturer's instructions. The SRA was performed with platelet rich plasma (PRP) as previously described (hereafter referred to as PRP-SRA)⁷ and was considered positive if there was <5% 14C serotonin release after patient plasma was added to platelets in the absence of heparin and >20% release after addition of 0.1 or 0.5 U/ml of heparin.

The KKO-I assay was performed as previously described in Sachais et al, Blood, 2012; 120(5): 1137-42. Briefly, Immulon 4 HBx 96-well plates (Thermo Fisher Scientific, Waltham, MA) coated with PF4 and heparin (Sagent Pharmaceuticals, Schaumburg, IL) were incubated with human plasma (1:50 dilution) for 30 minutes at 37°C followed by incubation with KKO for an additional 10 minutes at 37°C. Recombinant PF4 was expressed in Drosophila Schneider 2 cells and purified as previously described.⁶ KKO binding was measured as absorbance at 405 nm (A405) after incubation with HRP-conjugated goat anti-mouse IgG-Fc (Jackson ImmunoResearch Laboratories, West Grove, PA) and the HRP substrate ABTS (Roche Applied Science, Penzberg, Germany). Absorbance was measured with a SpectraCount plate reader (Packard BioScience, Waltham, MA). Data are presented as % inhibition of KKO binding, calculated as [(A405ₘₐₓ - A405ₚₐₜᵢₑₙₜ)/A405ₘₐₓ] × 100 with A405ₘₐₓ set in the absence of plasma. "0%" represents no inhibition of KKO binding and 100% represents complete inhibition.

The DT40-luc assay was performed as described in the above examples and in Sachais et al, Blood, 2012; 199(25):5955-5962. In brief, DT40 chicken B cells were transiently transfected to express human FcγRIIA (pEF6-FcγRIIA) as well as a reporter molecule (NFATLuc), which consists of the luciferase gene under control of the IL-2 promoter. On the day following transfection, the resultant DT40-luc cells were placed in 96-well culture plates. PF4/heparin complexes were formed first by incubating recombinant PF4 with heparin for 15 minutes at 37°C, followed by addition of patient plasma (1:800 final dilution). The PF4/heparin/plasma mixtures were then added to the cells for 6 hours at 37°C in an atmosphere containing 5% CO2. Plates were frozen at -80°C to terminate the activation reaction. To measure activation, cells were thawed and lysed with 5X Passive Lysis Buffer (Promega, Madison, WI) for 15 min. Luciferase activity was measured on a Berthold (Pforzheim, Germany) MultiLumat LB 9506 Luminometer (10 sec readings) using Luciferase Assay Reagent (Promega) following the manufacturer's instructions. Data are reported as the ratio of luciferase signal induced by patient plasma relative to the absence of plasma (fold-basal). Normalization of luciferase signal in the absence of plasma was performed with each transfection to account for potential differences in transfection efficiency.

We determined the 4Ts score for each subject by retrospective chart review to estimate the clinical likelihood of HIT at the time of laboratory testing. The investigator performing 4Ts scoring (AC) was blinded to the results of all HIT laboratory assays. Investigators performing the KKO-I and DT40-luc assays (AHR, JLH) were blinded to the 4Ts score and the results of the PRP-SRA and the polyspecific and IgG-specific PF4/heparin ELISA. The protocol was approved by the University of Pennsylvania institutional review board.

HIT was defined as the combination of an intermediate or high probability 4Ts score ≥4 and a positive PRP-SRA. The performance of the polyspecific ELISA, IgG-specific ELISA, KKO-I, and DT40-luc assay were evaluated with respect to this reference standard by receiver-operating characteristic (ROC) analysis. Areas under the ROC curves (AUCs) were calculated and compared by the DeLong method for correlated samples. Analyses were carried out using GraphPad Prism 5 (GraphPad Software, La Jolla, CA) and Analyse-it (Analyse-it Software, Leeds, UK). A p-value <0.05 was considered statistically significant.

### EXAMPLE 7: Results

Plasma samples from 58 subjects were studied. Each of the 21 subjects tested positive by PRP-SRA had a 4Ts score ≥4 and thus met the prespecified criteria for HIT. Plasma samples from 37 subjects tested negative by PRP-SRA and were considered negative for HIT. Demographic and clinical characteristics of HIT-positive and HIT-negative subjects are summarized in Table 3. The median 4Ts score was significantly greater in HIT-positive subjects (6 vs. 4, p<0.0001), though 21 (57%) HIT-negative subjects had an intermediate or high probability (≥4) score, highlighting the limited 3 specificity of clinical diagnosis. More patients in the HIT-positive group were on the cardiovascular surgical service (66.7% vs. 37.8%) and received treatment for HIT (90.5% vs. 67.6%), though these differences did not meet statistical significance (p=0.06 for both comparisons). Other demographic and clinical features were similar between the two groups (Table 3).

**Table 3 Demographic and clinical characteristics of HIT-positive and HIT-negative subjects.**

| **Characteristic** | **HIT-positive** (**n=21**) | **HIT-negative (n=37)** | **p-value** |
|---|---|---|---|
| Age: mean (range) | 66 (39-89) | 63 (39-80) | 0.47 |
| Female gender: n (%) | 9 (42.9) | 19(51.4) | 0.59 |
| Race: n (%) | | | 0.39 |
| White | 16 (76.2) | 23 (62.2) | |
| Black | 1 (4.8) | 6 (16.2) | |
| Other/Unknown | 4 (19.0) | 8 (21.6) | |
| Patient population: n (%) | | | |
| Cardiovascular surgery | 14 (66.7) | 14 (37.8) | 0.06 |
| Surgery (non-cardiovascular) | 5 (23.8) | 9 (24.3) | |
| Medicine | 2 (9.5) | 13 (35.1) | |
| Other | 0 | 1 (2.7) | |
| Setting: n (%) | | | 0.22 |
| Tertiary care hospital | 18 (85.7) | 26 (70.3) | |
| Community hospital | 3 (14.2) | 11 (29.7) | |
| Platelet count × 10⁹ /L: median (range)* | 50 (19-139) | 69 (3-883) | 0.29 |
| Recognized thrombosis: n (%)* | 14 (66.7) | 20 (54.1) | 0.41 |
| 4Ts score: median (interquartile range) | 6 (5-7) | 4 (3-5) | <0.0001 |
| Received treatment for HIT: n (%) | 19 (90.5) | 25 (67.6) | 0.06 |

| | | | |
|---|---|---|---|
| *At time of HIT laboratory testing | | | |

All 58 patient samples were tested using the polyspecific ELISA, IgG-specific ELISA, and DT40-luc. One HIT-positive and one HIT-negative sample were not tested by KKO-I due to insufficient sample volume. The ability of each assay to discriminate HIT-positive from HIT-negative subjects is shown in Figure 2 (panels A-D, 10 respectively). Consistent with previous studies, the mean OD was higher among HIT-positive than HIT-negative subject samples by both polyspecific (2.26 vs. 1.37, p<0.0001) and IgG-specific ELISA (1.86 vs. 1.14, p=0.0004). However, significant overlap in OD values among HIT-positive and HIT-negative subjects was observed with both assays (Figure 2A-B), underscoring their limited capacity to discriminate cell-activating (and presumably pathogenic) from non-activating PF4/heparin antibodies at the level of the individual patient.

KKO-I and DT40-luc showed better diagnostic discrimination than the commercially available ELISAs (Figure 2C-D). HIT-positive plasma samples exhibited significantly greater mean inhibition of KKO binding (78.9% vs. 26.0%, p<0.0001) (Figure 2C) and induced significantly greater luciferase activity (3.14-fold basal vs. 0.96-fold basal, p<0.0001) than HIT-negative samples (Figure 2D).

ROC curves for each assay are shown in Figure 3. The AUC for KKO-I (0.93, 95% CI 0.85-1.00) was significantly greater than the AUC for the polyspecific (0.82, 0.70-0.95; p=0.020) and IgG-specific (0.76, 0.62-0.90; p=0.0044) ELISAs, but not the DT40-luc (0.89, 0.79-0.99; p=0.40). The AUC for DT40-luc was significantly greater than the AUC for the IgG-specific (p=0.046), but not the polyspecific ELISA (p=0.28).

Table 4 shows the sensitivity/specificity pairs at the most northwest point on the ROC curve (the point at which sensitivity and specificity are optimized) for each assay. The cut-offs associated with these points are denoted by dashed horizontal lines in Figure 2. At a cut-off of 66%, the sensitivity and specificity of KKO-I was 0.90 and 0.92, respectively, and correctly classified 91% (51/56) of samples with respect to the reference standard. A lower cut-off of 50% improved sensitivity (0.95) at the cost of reduced specificity (0.72). The sensitivity and specificity of DT40-luc at a cut-off of 1.6-fold basal was 0.81 and 0.95, respectively, and was associated with correct classification of 88% (51/58) of samples. The operating characteristics of KKO-I and DT40-luc compared favorably with those of the polyspecific and IgG-specific ELISA. At cut-offs of 1.72 OD and 1.70 OD, the sensitivity of the latter assays was 0.76 and 0.67 and the specificity 0.78 and 0.76, respectively (Table 4). At these optimized cut-offs, the polyspecific ELISA correctly classified 78% (45/58) and the IgG-specific ELISA 72% (42/58) of samples.

**Table 4. Operating characteristics of the assays at the most northwest point on the receiver-operating characteristic curve.**

| **Assay** | **Cut-off** | **Sensitivity (95% CI)** | **Specificity (95% CI)** |
|---|---|---|---|
| Polyspecific ELISA | 1.72 OD | 0.76 (0.53-0.92) | 0.78 (0.62-0.90) |
| IgG-specific ELISA | 1.70 OD | 0.67 (0.43-0.85) | 0.76 (0.59-0.88) |
| KKO-I | 66% | 0.90 (0.68-0.99) | 0.92 (0.78-0.98) |
| DT40-luc | 1.6-fold basal | 0.81 (0.58-0.95) | 0.95 (0.82-0.99) |
| Combinatorial | KKO-I ≥ 75% or DT40-luc ≥ 2-fold basal | 0.95 (0.74-1.00) | 0.94 (0.80-0.99) |

Combinatorial analysis showed that the performance of KKO-I and DT40-luc could be further improved when both assays were integrated within a single diagnostic algorithm. A combinatorial strategy in which a sample was considered positive if it demonstrated either KKO-I ≥ 75% or DT40-luc ≥ 2.0-fold basal would have resulted in correct classification of 55 of the 58 patients and a sensitivity and specificity of 0.95 and 0.94, respectively (Table 4). Conjunctive combinatorial strategies (i.e. Boolean operations using "AND" rather than "OR") did not result in improved performance.

Because the polyspecific PF4/heparin ELISA has very high *negative* predictive 3,10 value for HIT, we chose to confine our study cohort to the more diagnostically challenging patients who test *positive* by polyspecific ELISA (OD ≥ 0.4). Nevertheless, samples from 9 additional subjects with suspected HIT that tested negative in the polyspecific ELISA were analyzed as negative controls. As expected, all 9 samples showed negligible inhibition of KKO binding (range: -16.1% to 11.9%) and luciferase activity (0.75-fold basal to 1.37-fold basal) (data not shown).

### EXAMPLE 8: Discussion

Binding of KKO to immobilized PF4/heparin is inhibited to a greater extent by PRP-SRA-positive plasma than by plasma from patients with non-cell-activating anti-5 PF4/heparin antibodies. The KKO-I assay was designed to leverage this property of KKO, which possesses *in vitro* and *in vivo* platelet-activating activity, for the purpose of discriminating cell-activating and potentially pathogenic HIT antibodies from their non-pathogenic counterparts. In the present study, plasma from HIT-positive subjects demonstrated significantly greater mean inhibition of KKO binding than HIT-negative plasma (78.9% vs. 26.0%, p<0.0001) by KKO-I (Figure 2C). These findings suggest that human cell-activating antibodies in HIT plasma bind to epitopes on PF4 that overlap to a greater extent with KKO's binding site(s) than epitopes recognized by non-platelet activating antibodies, such as the isotype-matched monoclonal anti-PF4 antibody RTO⁵, and point to epitope specificity as a potential major determinant of anti-PF4/heparin antibody pathogenicity.

KKO-I is not the only diagnostic assay for HIT to make use of KKO. HemosIL HIT-Ab_{(PF4-H)} (Instrumentation Laboratory, Bedford, MA) is a latex particle enhanced immunoturbidimetric assay in which agglutination of KKO-coated latex beads to PF4/polyvinylsulfonate complexes in solution is inhibited in the presence of human anti-PF4/heparin antibodies. Like the polyspecific PF4/heparin ELISA, HemosIL HIT-Ab(PF4-H) has high sensitivity but appears to be limited in its capacity to discriminate cell-activating from non-pathogenic antibodies. The assay was studied in a cohort of 102 subjects with suspected HIT, in which HIT was defined as an intermediate or high clinical suspicion coupled with a positive aggregometry-based functional assay. All 17 samples meeting this definition tested positive by HemosIL HIT-Ab_{(PF4-H)} (sensitivity 100%), but 16 additional HIT-negative samples also tested positive (specificity 81%).11 Although KKO-I and HemosIL HIT-Ab_{(PF4-H)} have not been compared head-to-head, our data suggest that the former may be more specific for platelet-activating antibodies. The reasons for this apparent discrepancy in specificity are unknown, but may relate to differences in the geometry and kinetics of KKO. The spatial orientation and intermolecular organization of KKO on the latex bead may permit steric inhibition by a higher proportion of anti-PF4/heparin antibodies of diverse epitope specificities compared with more stringent requirements to compete with the same antibody in solution. In addition, the epitope(s) recognized by KKO on complexes of PF4/polyvinylsulfonate in solution may differ sufficiently from those on immobilized complexes of PF4/heparin that nonpathogenic antibodies can compete effectively. Better understanding of these differences may lead to more detailed characterization of the pathogenic epitopes in HIT, which in turn, could be used to further improve assay specificity.

Previous work has shown that FcγRIIa is required for the platelet activation central to the pathogenesis of HIT¹² . To obviate the need for fresh, reactive, donor platelets, we developed a DT40 (chicken B lymphocyte) cell line transfected with human FcγRIIa coupled to a luciferase reporter to measure cellular activation by HIT antibodies⁶. In this platelet-free system (DT40-luc), plasma from HIT-positive subjects induced significantly greater luciferase activity than plasma from HIT-negative subjects (3.14-fold basal vs. 0.96-fold basal, p<0.0001) (Figure 2D). Thus, this assay recapitulates the salient requirements to induce HIT, namely PF4-heparin-IgG complexes capable of activating cells via FcγRIIa,¹²⁻¹⁵ without the need for platelet-specific antigens. This is an important feature in light of emerging evidence that activation of other cell types (e.g. endothelial cells, monocytes) may contribute to the prothrombotic phenotype of HIT through this and other Fcγ receptor.¹⁶⁻¹⁸

In the current study, KKO-I and DT40-luc exhibited better discrimination than a commercially available polyspecific and IgG-specific ELISA (Figure 2). At the most northwest point on their respective ROC curves (Figure 3), the sensitivity and specificity of the novel assays were superior to those of the commercial tests (Table 4). The sensitivity of the polyspecific and IgG-specific ELISA is lower than that reported in other studies (0.95-1.00), ¹⁹primarily because we used the most northwest point on the ROC curve in our analysis rather than the manufacturer-recommended cut-off of 0.40. The specificity of all four assays would likely have been enhanced had samples from patients testing negative by polyspecific PF4/heparin ELISA, a group that typically comprises 70% to 90% of a reference laboratory's test population¹⁹, been included in the study cohort.

Our findings have potentially important clinical implications. The diagnosis of HIT is a high stakes enterprise. Delays in diagnosis and institution of appropriate therapy are associated with an initial 6.1% daily risk of thromboembolism, amputation, and death. ²⁰Misdiagnosis, conversely, may result in unnecessary exposure of thrombocytopenic patients without HIT to direct thrombin inhibitors and their attendant 1% daily risk of major hemorrhage^{21,22}. The latter is a particularly prevalent problem in current practice²³, as highlighted by the observation that two-thirds of the 37 HIT-negative subjects in our study nonetheless received treatment for HIT (Table 3) with potential bleeding complications and unlikely benefit.

The limited specificity of currently available immunoassays and the limited availability of more specific functional assays contribute to the problem of overdiagnosis and unnecessary treatment. Immunoassays such as the polyspecific PF4/heparin ELISA are simple to perform and widely used, but are unable to discriminate cell-activating and potentially pathogenic from non-pathogenic antibodies (Figure 2A). Depending on the patient population, only 28% to 59% of samples testing positive by polyspecific ELISA also test positive by a more specific functional assay. ²⁴⁻²⁷ The OD of a positive ELISA is a helpful, but relatively crude predictor of pathogenicity (Figures 2A-B). ¹⁰Modifications of the PF4/heparin ELISA that detect only antibodies of the IgG class (e.g. IgG-specific ELISA) or antibodies inhibited by excess heparin (e.g. high-dose heparin confirmatory test) enhance specificity, ^{24,27,28} but false-positive results remain common with these approaches and reductions in sensitivity have also been reported^{19,29}.

KKO-I and DT40-luc demonstrated better diagnostic discrimination than a commercially available polyspecific and IgG-specific ELISA in the present study (Figure 2, Table 4) and hold promise for improving the specificity of laboratory diagnosis and curtailing the current trend of overdiagnosis. Of the 37 HIT-negative subjects in our study, all would have been misdiagnosed with (and potentially treated for) HIT based on the manufacturer-recommended polyspecific ELISA OD cut-off of 0.40. Even use of an optimized cut-off corresponding to the most northwest point on the ROC curve would have resulted in misclassification of 13 of 58 (22%) subjects by polyspecific ELISA and 16 of 58 (28%) by IgG-specific ELISA. In contrast, KKO-I and DT40-luc correctly classified 33 of 36 (92%) and 35 of 37 (95%) HIT-negative patients, respectively, suggesting the potential for these novel assays to curb the current problem of overdiagnosis and unnecessary treatment.

Functional assays for HIT such as the SRA and the heparin-induced platelet activation (HIPA) test are sensitive and more specific than commercial immunoassays, but are impracticable for most clinical laboratories due to the need for fresh reactive donor platelets, radioisotope (for the SRA), and meticulous technique and platelet aggregometry (for the HIPA). ^{7,30}KKO-I and DT40-luc may represent more feasible options for laboratories seeking to improve the specificity of the testing they offer. As a modification of the PF4/heparin ELISA, KKO-I could be relatively easily adopted by laboratories that currently perform the former assay. DT40-luc is a test of cellular activation in which donor platelets are replaced by a cell-line that can be stored at -80°C and retrieved as needed for testing. The endpoint for cellular activation is luciferase activity, which can be measured by a standard spectrophotometer without need for radioactivity or platelet aggregometry.

Several limitations of our study deserve mention. First, the study population was relatively small and was recruited from two hospitals within a single health system. Validation in a larger multicenter study is required. Second, the PRP-SRA employed in the current study⁷ differs from the more widely used washed platelet SRA.³¹ PRP-based aggregometry assays may be less specific than washed platelet functional assays,³² though SRAs utilizing PRP and washed platelets have not been directly compared. Third, there is no universally accepted gold standard for HIT. In a recent meta-analysis, a 4Ts 33 score ≥4 was associated with a positive predictive value of only 22%. Positive results by SRA in the absence of clinical HIT may also occur, particularly after cardiac 34 surgery. To overcome these limitations in the positive predictive value of the 4Ts and SRA, we used a reference standard (4Ts score ≥4 and positive PRP-SRA) incorporating both clinical and laboratory criteria, as recommended by an international expert 35 consensus panel. Nevertheless, misclassification of some subjects in our study cannot be excluded.

With this caveat in mind, two subjects that met the prespecified definition of HIT tested negative by KKO-I at a cut-off of 66% (Figure 2C). One was a 43-year-old woman who developed thrombocytopenia, lower extremity deep vein thrombosis, and pulmonary embolism following resection of locally advanced ovarian cancer. Her 4Ts score was 7 (high probability) and her platelet count normalized rapidly on argatroban. Her plasma showed only 1% inhibition by KKO-I and 0.81-fold basal activation by DT40-luc. Interestingly, weakly positive results were obtained by polyspecific ELISA, IgG-specific ELISA, and PRP-SRA (0.67, 0.41, and 43%, respectively). Values in this range have been reported to occur in the absence of clinical HIT, 10,36 suggesting that the patient may have been misclassified by the reference standard rather than by KKO-I. The second patient was a 72-year-old man who developed thrombocytopenia, lower extremity deep vein thrombosis, and necrotic skin lesions at heparin injection sites following percutaneous repair of a celiac artery aneurysm (4Ts score = 7). Thrombocytopenia resolved with cessation of heparin and initiation of argatroban. Results of the polyspecific ELISA, IgGspecific ELISA, and PRP-SRA were 1.32, 0.97, and 93%, respectively. Although the patient tested negative by KKO-I (51% inhibition), his plasma induced strong luciferase activity (6.45-fold basal) by the DT40-luc assay. This laboratory profile suggests the possibility of an uncommon variant of HIT in which cell-activating antibodies recognize complexes of heparin and cationic proteins other than PF4 such as interleukin-8 or neutrophil-activating peptide-2. ³⁷Such antibodies might be detectable using functional assays (e.g. DT40-luc), but not by PF4/heparin immunoassays (e.g. KKO-I).

As suggested by this second case in which discrepant results were obtained by KKO-I and DT40-luc, a combinatorial approach may yield better performance than use of either assay alone. Indeed, an integrated algorithm in which a sample was considered positive if it demonstrated either KKO-I ≥ 75% or DT40-luc ≥ 2.0-fold basal would have resulted in correct classification of 55 of the 58 patients in our study and a sensitivity and specificity of 0.95 (0.74-1.00) and 0.94 (0.80-0.99), respectively (Table 4). The operating characteristics of such an approach require validation in an independent test population.

In summary, we describe a novel immunoassay (KKO-I) and a novel functional assay (DT40-luc) for the diagnosis of HIT. KKO-I and DT40-luc discriminated cell-activating and potentially pathogenic antibodies from non-activating antibodies more effectively than two commercially available ELISAs in a small cohort of patients with suspected HIT. These assays are simple to perform; do not require donor platelets, radioactivity, or platelet aggregometry; and hold promise for improving the specificity and feasibility of HIT laboratory testing. Validation of KKO-I and DT40-luc in a larger prospective study is underway. While the invention has been described with reference to particular embodiments, it will be appreciated that modifications can be made. Such modifications are intended to fall within the scope of the appended claims.

### References:

1. Amiral J, Bridey F, Dreyfus M, Vissoc AM, Fressinaud E, Wolf M, Meyer D. Platelet factor 4 complexed to heparin is the target for antibodies generated in heparin-induced thrombocytopenia. Thromb Haemost. 1992;68(1):95-96.
2. Rauova L, Poncz M, McKenzie SE, et al. Ultralarge complexes of PF4 and heparin are central to the pathogenesis of heparin-induced thrombocytopenia. Blood. 2005;105(1):131-138.
3. Cuker A, Cines DB. How I treat heparin-induced thrombocytopenia. Blood. 2012;119(10):2209-2218.
4. Arepally GM, Kamei S, Park KS, et al. Characterization of a murine monoclonal antibody that mimics heparin-induced thrombocytopenia antibodies. Blood. 2000;95(5):1533-1540.
5. Sachais BS, Litvinov RI, Yarovoi SV, et al. Dynamic antibody-binding properties in the pathogenesis of HIT. Blood. 2012;120(5):1137-1142.
6. Sachais BS, Rux AH, Cines DB, et al. Rational design and characterization of platelet factor 4 antagonists for the study of heparin-induced thrombocytopenia. Blood. 2012;119(25):5955-5962.
7. Cines DB, Kaywin P, Bina M, Tomaski A, Schreiber AD. Heparin-associated thrombocytopenia. N Engl JMed. 1980;303(14):788-795.
8. Lo GK, Juhl D, Warkentin TE, Sigouin CS, Eichler P, Greinacher A. Evaluation of pretest clinical score (4 T's) for the diagnosis of heparin-induced thrombocytopenia in two clinical settings. J Thromb Haemost. 2006;4(4):759-765.
9. DeLong ER, DeLong DM, Clarke-Pearson DL. Comparing the areas under two or more correlated receiver operating characteristic curves: a nonparametric approach. Biometrics. 1988;44(3):837-845.
10. Warkentin TE, Sheppard JI, Moore JC, Sigouin CS, Kelton JG. Quantitative interpretation of optical density measurements using PF4-dependent enzyme-immunoassays. J Thromb Haemost. 2008;6(8):1304-1312.
11. Legnani C, Cini M, Pili C, Boggian O, Frascaro M, Palareti G. Evaluation of a new automated panel of assays for the detection of anti-PF4/heparin antibodies in patients suspected of having heparin-induced thrombocytopenia. Thromb Haemost. 2010;104(2):402-409.
12. Reilly MP, Taylor SM, Hartman NK, et al. Heparin-induced thrombocytopenia/thrombosis in a transgenic mouse model requires human platelet factor 4 and platelet activation through FcgammaRIIA. Blood. 2001;98(8):2442-2447.
13. Visentin GP, Ford SE, Scott JP, Aster RH. Antibodies from patients with heparin-induced thrombocytopenia/thrombosis are specific for platelet factor 4 complexed with heparin or bound to endothelial cells. J Clin Invest. 1994;93(1):81-88.
14. Greinacher A, Pötzsch B, Amiral J, Dummel V, Eichner A, Mueller-Eckhardt C. Heparin-associated thrombocytopenia: isolation of the antibody and characterization of a multimolecular PF4-heparin complex as the major antigen. Thromb Haemost. 1994;71 (2):247-251.
15. Kelton JG, Smth JW, Warkentin TE, Hayward CP, Denomme GA, Horsewood P. Immunoglobulin G from patients with heparin-induced thrombocytopenia binds to a complex of heparin and platelet factor 4. Blood. 1994;83(11):3232-3239.
16. Blank M, Schoenfeld Y, Tavor S, et al. Anti-platelet factor 4/heparin antibodies from patients with heparin-induced thrombocytopenia provoke direct activation of microvascular endothelial cells. Int Immunol. 2002;14(2):121-129.
17. Rauova L, Hirsch JD, Greene TK, et al. Monocyte-bound PF4 in the pathogenesis of heparin-induced thrombocytopenia. Blood. 2010;116(23):5021-5031.
18. Kasthuri RS, Glover SL, Jonas W, et al. PF4/heparin-antibody complex induced monocyte tissue factor expression and release of tissue factor positive microparticles by activation of FcγRI. Blood. 2012;119(22):5285-5293.
19. Cuker A, Ortel TL. ASH evidence-based guidelines: is the IgG-specific anti-PF4/heparin ELISA superior to the polyspecific ELISA in the laboratory diagnosis of HIT? Hematology Am Soc Hematol Educ Program. 2009;250-252.
20. Greinacher A, Eichler P, Lubenow N, Kwasny H, Luz M. Heparin-induced thrombocytopenia with thromboembolic complications: meta-analysis of 2 prospective trials to assess the value of parenteral treatment with lepirudin and its therapeutic aPTT range. Blood. 2000;96(3):846-851.
21. Lubenow N, Eichler P, Lietz T, Greinacher A; Hit Investigators Group. Lepirudin in patients with heparin-induced thrombocytopenia - results of the third prospective study (HAT-3) and a combined analysis of HAT-1, HAT-2, and HAT-3. J Thromb Haemost. 2005;3(11):2428-2436.
22. Lewis BE, Wallis DE, Hursting MJ, Levine RL, Leya F. Effects of argatroban therapy, demographic variables, and platelet count on thrombotic risks in heparin-induced thrombocytopenia. Chest. 2006;129(6): 1407-1416.
23. Cuker A. Heparin-induced thrombocytopenia (HIT) in 2011: an epidemic of overdiagnosis. Thromb Haemost. 2011;106(6):993-994.
24. Juhl D, Eichler P, Lubenow N, Strobel U, Wessel A, Greinacher A. Incidence and clinical significance of anti-PF4/heparin antibodies of the IgG, IgM, and IgA class in 755 consecutive patient samples referred for diagnostic testing for heparin-induced thrombocytopenia. Eur J Haematol. 2006;76(5):420-426.
25. Lo GK, Juhl D, Warkentin TE, Sigouin CS, Eichler P, Greinacher A. Evaluation of pretest clinical score (4 T's) for the diagnosis of heparin-induced thrombocytopenia in two clinical settings. J Thromb Haemost. 2006;4(4):759-765.
26. Pouplard C, Gueret P, Fouassier M, Ternisien C, Trossaert M, Régina S, Gruel Y. Prospective evaluation of the '4Ts' score and particle gel immunoassay specific to heparin/PF4 for the diagnosis of heparin-induced thrombocytopenia. J Thromb Haemost. 2007;5(7):1373-1379.
27. Bakchoul T, Giptner A, Najaoui A, Bein G, Santoso S, Sachs UJ. Prospective evaluation of PF4/heparin immunoassays for the diagnosis of heparin-induced thrombocytopenia. J Thromb Haemost. 2009;7(8):1260-1265.
28. Whitlatch NL, Kong DF, Metjian AD, Arepally GM, Ortel TL. Validation of the high-dose heparin confirmatory step for the diagnosis of heparin-induced thrombocytopenia. Blood. 2010; 116(10):1761-1766.
29. Althaus K, Strobel U, Warkentin TE, Greinacher A. Combined use of the high heparin step and optical density to optimize diagnostic sensitivity and specificity of an anti-PF4/heparin enzyme-immunoassay. Thromb Res. 2011;128(3):256-260.
30. Greinacher A, Michels I, Kiefel V, Mueller-Eckhardt C. A rapid and sensitive test for diagnosing heparin-associated thrombocytopenia. Thromb Haemost. 1991;66(6):734-736.
31. Sheridan D, Carter C, Kelton JG. A diagnostic test for heparin-induced thrombocytopenia. Blood. 1986;67(1):27-30.
32. Warkentin TE. Heparin-induced thrombocytopenia in the ICU: a transatlantic perspective. Chest. 2012;142(4):815-816.
33. Cuker A, Gimotty PA, Crowther MA, Warkentin TE. Predictive value of the 4Ts scoring system for heparin-induced thrombocytopenia: a systematic review and meta-analysis. Blood. 2012. In press.
34. Bauer TL, Arepally G, Konkle BA, et al. Prevalence of heparin-associated antibodies without thrombosis in patients undergoing cardiopulmonary bypass surgery. Circulation. 1997;95(5): 1242-1246.
35. Warkentin TE, Greinacher A, Gruel Y, Aster RH, Chong BH; scientific and standardization committee of the international society on thrombosis and haemostasis. Laboratory testing for heparin-induced thrombocytopenia: a conceptual framework and implications for diagnosis. J Thromb Haemost. 2011;9(12):2498-2500.
36. Warkentin TE, Levine MN, Hirsh J, Horsewood P, Roberts RS, Gent M, Kelton JG. Heparin-induced thrombocytopenia in patients treated with low-molecular-weight heparin or unfractionated heparin. N Engl J Med. 1995;332(20):1330-1335.
37. Amiral J, Marfaing-Koka A, Wolf M, et al. Presence of autoantibodies to interleukin-8 or neutrophil-activating peptide-2 in patients with heparin-associated thrombocytopenia. Blood. 1996;88(2):410-416.

## Claims

1. A method for identifying antibodies or fragments thereof that activate heparin-induced thrombocytopenia (HIT) comprising
(a) contacting a hematopoietic cell or cell line that comprises, in operative association, the platelet receptor FcγRIIA under the control of a suitable promoter, and a reporter construct comprising a reporter gene under the control of a promoter and transcription factor, which transcription factor is regulated downstream of the signaling cascade of activated FcγRIIA, with
i. a test sample from a mammalian subject;
ii. a platelet factor 4 (PF4), a wild-type or variant of PF4 or a functional fragment thereof; and
iii. heparin or a heparin-like molecule or a heparinoid molecule; and
(b) detecting or measuring the level of reporter gene expression.

2. The method according to claim 1, wherein, when the test sample contains an HIT-activating antibody or fragment, the antibody and PF4-heparin complexes in contact with the cell or cell line activate FcγRIIA and its signaling cascade, thereby generating downstream products that activate the transcription factor and promoter of the reporter construct, resulting in an increase in reporter gene expression; and wherein when the test sample does not contain an HIT-activating antibody or fragment, FcγRIIA and its signaling cascade are not activated, and no increase in reporter gene expression occurs.

3. The method according to claim 1 or 2, wherein the mammalian subject is a human.

4. The method according to any one of claims 1 to 3, wherein the test sample is blood, plasma, serum, or cell eluate.

5. The method according to any one of claims 1 to 4, further comprising comparing the reporter gene expression in the cells containing the test sample or test molecule to a reference selected from:
a basal level of reporter expression generated by the cells in the absence of an HIT-activating antibody or in the presence of a negative control that does not activate FcγRIIA in the cells;
a level of reporter expression generated in the presence of a positive control that activates FcγRIIA in the cells; and
a level of reporter expression generated in the absence of a test sample or test molecule.

6. The method according to claim 5, wherein the negative control is human plasma from a subject without HIT, a non-antigenic PF4 that cannot form ultra large complexes with heparin, or an anti-coagulant that does not form ultra large complexes with PF4.

7. The method according to any one of claims 1 to 6, wherein the PF4 is human PF4.

8. The method according to any one of claims 1 to 7, wherein the reporter gene expression is increased in a heparin and PF4-dependent manner.

9. A stable hematopoietic cell line for use in performing the method of claim 1, the cell line comprising, in operative association, the platelet receptor FcγRIIA under the control of a suitable promoter and a reporter construct comprising a reporter gene under the control of a promoter and transcription factor, which are activated by downstream products of the signaling cascade of activated FcγRIIA.

10. The method or cell line according to any one of claims 1 to 9, wherein the reporter construct comprises a reporter gene under the control of a composite promoter comprising the nuclear factor of activated T-cells (NFAT)-AP-1 sites from the IL-2 promoter, which requires both Ca²⁺ and map kinase (MAPK) generated by the signaling cascade of activated FcγRIIA to activate the NFAT transcription factor, resulting in increased expression of the reporter.

11. The method or cell line according to any of claims 1 to 10, wherein the hematopoietic cell is co-transfected with a first plasmid expressing FcγRIIA under the control of a suitable promoter, and a second plasmid carrying the reporter construct.

12. The method or cell line according to any one of claims 1 to 11, wherein hematopoietic cell or cell line is a B lineage cell or cell line or a T lineage cell or cell line.

13. The method or cell line according to any of claims 1 to 12, wherein the FcγRIIA coding sequence is a human sequence.

14. The method or cell line according to any of claims 1 to 13, wherein the FcγRIIA coding sequence is under the control of the human EF-1α promoter.

15. The method or cell line according to claim 10, wherein NFAT is one of the calcium sensitive NFATc1, NFATc2, NFATc3, or NFATc4.

## Patentansprüche

1. Verfahren zur Identifizierung von Antikörpern oder Fragmenten davon, welche Heparin-induzierte Thrombozytopenie (HIT) aktivieren, umfassend
(a) Kontaktieren einer hämatopoietischen Zelle oder Zelllinie, welche in Wirkverbindung den von einem geeigneten Promotor gesteuerten Thrombozyten-Rezeptor FcγRIIA sowie ein Reporterkonstrukt umfasst, welches ein von einem Promotor und einem Transkriptionsfaktor gesteuertes Reportergen umfasst, wobei der Transkriptionsfaktor stromabwärts der Signalkaskade des aktivierten FcγRIIA reguliert wird, mit
i. einer von einem Säuger stammenden Testprobe,
ii. einem Plättchenfaktor 4 (PF4), einem Wildtyp oder einer Variante von PF4 oder einem funktionellen Fragment davon und
iii. Heparin oder einem heparinartigen Molekül oder einem Heparinoid-Molekül und
(b) Nachweisen oder Messen des Reportergen-Expressionslevels.

2. Verfahren nach Anspruch 1, wobei die die Zelle oder Zelllinie kontaktierenden Antikörper- und PF4-Heparin-Komplexe FcγRIIA und dessen Signalkaskade aktivieren, wenn die Testprobe einen HIT aktivierenden Antikörper oder ein solches Fragment enthält, wodurch Folgeprodukte erzeugt werden, welche den Transkriptionsfaktor und den Promotor des Reporterkonstrukts aktivieren, woraus eine Steigerung der Reportergen-Expression resultiert, und wobei FcγRIIA und dessen Signalkaskade nicht aktiviert werden und keine Steigerung der Reportergen-Expression auftritt, wenn die Testprobe keinen HIT aktivierenden Antikörper oder ein solches Fragment enthält.

3. Verfahren nach Anspruch 1 oder 2, wobei es sich bei dem Säuger um einen Menschen handelt.

4. Verfahren nach einem der Ansprüche 1 bis 3, wobei es sich bei der Testprobe um Blut, Plasma, Serum oder Zell-Eluat handelt.

5. Verfahren nach einem der Ansprüche 1 bis 4, ferner umfassend ein Vergleichen der Reportergen-Expression in den die Testprobe oder das Testmolekül enthaltenden Zellen mit einer Referenz, welche ausgewählt ist aus:
einem Basallevel der Reporter-Expression, welcher von den Zellen in Abwesenheit eines HIT aktivierenden Antikörpers oder in Gegenwart einer Negativkontrolle erzeugt wird, welche FcγRIIA in den Zellen nicht aktiviert,
einem Reporter-Expressionslevel, welcher in Gegenwart einer Positivkontrolle erzeugt wird, welche FcγRIIA in den Zellen aktiviert, und
einem Reporter-Expressionslevel, welcher in Abwesenheit einer Testprobe oder eines Testmoleküls erzeugt wird.

6. Verfahren nach Anspruch 5, wobei es sich bei der Negativkontrolle um Humanplasma von einer Testperson ohne HIT, ein nicht antigenes PF4, welches keine ultragroßen Komplexe mit Heparin bilden kann, oder um ein Antikoagulans handelt, welches keine ultragroßen Komplexe mit PF4 bildet.

7. Verfahren nach einem der Ansprüche 1 bis 6, wobei es sich bei dem PF4 um humanes PF4 handelt.

8. Verfahren nach einem der Ansprüche 1 bis 7, wobei die Reportergen-Expression in einer von Heparin und PF4 abhängigen Art gesteigert wird.

9. Stabile hämatopoietische Zelllinie zur Verwendung bei der Durchführung des Verfahrens nach Anspruch 1, wobei die Zelllinie in Wirkverbindung den von einem geeigneten Promotor gesteuerten Thrombozyten-Rezeptor FcγRIIA sowie ein Reporterkonstrukt umfasst, welches ein Reportergen umfasst, das von einem Promotor und einem Transkriptionsfaktor gesteuert wird, welche von Folgeprodukten der Signalkaskade des aktivierten FcγRIIA aktiviert werden.

10. Verfahren oder Zelllinie nach einem der Ansprüche 1 bis 9, wobei das Reporterkonstrukt ein Reportergen umfasst, welches von einem zusammengesetzten Promotor gesteuert wird, welcher den Kernfaktor aktivierter T-Zellen-(NFAT)-AP-1-Positionen des IL-2-Promotors umfasst, wobei jeweils von der Signalkaskade des aktivierten FcγRIIA erzeugtes Ca²⁺ und MAP-Kinase (MAPK) zum Aktivieren des NFAT-Transkriptionsfaktors benötigt werden, woraus eine gesteigerte Expression des Reporters resultiert.

11. Verfahren oder Zelllinie nach einem der Ansprüche 1 bis 10, wobei die hämatopoietische Zelle mit einem ersten Plasmid, welches von einem geeigneten Promotor gesteuertes FcγRIIA exprimiert, und einem zweiten, das Reporterkonstrukt tragenden Plasmid kotransfiziert wird.

12. Verfahren oder Zelllinie nach einem der Ansprüche 1 bis 11, wobei es sich bei der hämatopoietischen Zelle oder Zelllinie um eine B-Abstammungszelle oder -Zelllinie oder eine T- Abstammungszelle oder -Zelllinie handelt.

13. Verfahren oder Zelllinie nach einem der Ansprüche 1 bis 12, wobei es sich bei der FcγRIIA-Codierungssequenz um eine humane Sequenz handelt.

14. Verfahren oder Zelllinie nach einem der Ansprüche 1 bis 13, wobei die FcγRIIA-Codierungssequenz von dem humanen EF-1α-Promotor gesteuert wird.

15. Verfahren oder Zelllinie nach Anspruch 10, wobei es sich bei NFAT um eines der Calcium-sensitiven NFATc1, NFATc2, NFATc3 oder NFATc4 handelt.

## Revendications

1. Procédé pour identifier des anticorps ou des fragments de ceux-ci qui activent une thrombocytopénie induite par héparine (HIT) comprenant :
(a) la mise en contact d'une cellule ou lignée cellulaire hématopoïétique qui comprend, en association fonctionnelle, le récepteur de plaquettes FcγRIIA sous le contrôle d'un promoteur approprié, et une construction rapporteur qui comprend un gène rapporteur sous le contrôle d'un promoteur et facteur de transcription, lequel facteur de transcription est régulé en aval de la cascade de signalisation de FcγRIIA activé, avec :
i. un échantillon de test qui provient d'un sujet qui est un mammifère ;
ii. un facteur de plaquettes 4 (PF4), un PF4 sauvage ou variant ou un fragment fonctionnel de celui-ci ; et
iii. une molécule d'héparine ou similaire à l'héparine ou une molécule héparinoïde ; et
(b) la détection ou la mesure du niveau de l'expression du gène rapporteur.

2. Procédé selon la revendication 1, dans lequel, lorsque l'échantillon de test contient un anticorps d'activation de HIT ou un fragment de celui-ci, les complexes anticorps et PF4-héparine en contact avec la cellule ou lignée cellulaire activent FcγRIIA et sa cascade de signalisation, générant ainsi des produits en aval qui activent le facteur de transcription et promoteur de la construction rapporteur, ce qui résulte en une augmentation de l'expression du gène rapporteur ; et dans lequel, lorsque l'échantillon de test ne contient pas un anticorps d'activation de HIT ou un fragment de celui-ci, FcγRIIA et sa cascade de signalisation ne sont pas activés, et aucune augmentation de l'expression du gène rapporteur n'est observée.

3. Procédé selon la revendication 1 ou 2, dans lequel le sujet qui est un mammifère est un être humain.

4. Procédé selon l'une quelconque des revendications 1 à 3, dans lequel l'échantillon de test est du sang, du plasma, du sérum ou de l'éluat de cellules.

5. Procédé selon l'une quelconque des revendications 1 à 4, comprenant en outre la comparaison de l'expression du gène rapporteur dans les cellules contenant l'échantillon de test ou la molécule de test avec une référence qui est sélectionnée parmi :
un niveau de base de l'expression du rapporteur qui est générée par les cellules en l'absence d'un anticorps d'activation de HIT ou en présence d'un contrôle négatif qui n'active pas FcγRIIA dans les cellules ;
un niveau de l'expression du rapporteur qui est générée en présence d'un contrôle positif qui active FcγRIIA dans les cellules ; et
un niveau de l'expression du rapporteur qui est générée en l'absence d'un échantillon de test ou d'une molécule de test.

6. Procédé selon la revendication 5, dans lequel le contrôle négatif est du plasma humain qui provient d'un sujet sans HIT, un PF4 non antigénique qui ne peut pas former des complexes ultra-larges avec l'héparine, ou un anticoagulant qui ne forme pas de complexes ultralarges avec PF4.

7. Procédé selon l'une quelconque des revendications 1 à 6, dans lequel le PF4 est du PF4 humain.

8. Procédé selon l'une quelconque des revendications 1 à 7, dans lequel l'expression du gène rapporteur est augmentée d'une manière qui dépend de l'héparine et du PF4.

9. Ligne cellulaire hématopoïétique stable pour une utilisation pour la mise en oeuvre du procédé selon la revendication 1, la lignée cellulaire comprenant, en association fonctionnelle, le récepteur de plaquettes FcγRIIA sous le contrôle d'un promoteur approprié et une construction rapporteur qui comprend un gène rapporteur sous le contrôle d'un promoteur et facteur de transcription, lesquels sont activés par des produits en aval de la cascade de signalisation de FcγRIIA activé.

10. Procédé ou lignée cellulaire selon l'une quelconque des revendications 1 à 9, dans lequel ou laquelle la construction rapporteur comprend un gène rapporteur sous le contrôle d'un promoteur composite qui comprend les sites du facteur nucléaire de cellules T activées (NFAT)-AP-1 du promoteur IL-2, lequel nécessite à la fois du Ca²⁺ et de la MAP kinase (MAPK) générée par la cascade de signalisation du FcγRIIA activé afin d'activer le facteur de transcription NFAT, ce qui résulte en une expression augmentée du rapporteur.

11. Procédé ou lignée cellulaire selon l'une quelconque des revendications 1 à 10, dans lequel ou laquelle la cellule hématopoïétique est co-transfectée avec un premier plasmide qui exprime FcγRIIA sous le contrôle d'un promoteur approprié, et un second plasmide qui est porteur de la construction rapporteur.

12. Procédé ou lignée cellulaire selon l'une quelconque des revendications 1 à 11, dans lequel ou laquelle une cellule ou lignée cellulaire hématopoïétique est une cellule ou lignée cellulaire de lignée B ou une cellule ou lignée cellulaire de lignée T.

13. Procédé ou lignée cellulaire selon l'une quelconque des revendications 1 à 12, dans lequel ou laquelle la séquence codant pour FcγRIIA est une séquence humaine.

14. Procédé ou lignée cellulaire selon l'une quelconque des revendications 1 à 13, dans lequel ou laquelle la séquence codant pour FcγRIIA est sous le contrôle du promoteur EF-1α humain.

15. Procédé ou lignée cellulaire selon la revendication 10, dans lequel ou laquelle NFAT est l'un des facteurs sensibles au calcium NFATc1, NFATc2, NFATc3 ou NFATc4.
